# EUROPEAN PATENT SPECIFICATION

(11) **EP 0 993 301 B1**
(45) Date of publication and mention of the grant of the patent: **09.04.2003**
(21) Application number: 97940580.0
(22) Date of filing: 22.08.1997
(51) Int. Cl.: A61K 31/46, C07D 451/02, A61P 25/30, A61P 25/16

(54) **COCAINE RECEPTOR BINDING LIGANDS**
COCAIN-REZEPTOR BINDENDE LIGANDEN
LIGANDS LIANT DES RECEPTEURS DE COCAINE

(30) Priority: 22.08.1996 US 701503; 04.09.1996 US 706263; 25.03.1997 US 823563
(43) Date of publication of application: 19.04.2000
(73) Proprietor: RESEARCH TRIANGLE INSTITUTE, Research Triangle Park, NC 27709 (US)
(72) Inventor: KUHAR, Michael, J., Baltimore, MD 21209 (US); CARROLL, Frank, I., Durham, NC 27712 (US); BOJA, John, W., Baltimore, MD 21236 (US); LEWIN, Anita, H., Chapel Hill, NC 27515 (US); ABRAHAM, Philip, Cary, NC 27511 (US)
(74) Representative: Audier, Philippe André
(86) International application number: PCT/US97/14702
(87) International publication number: WO 98/007427

(56) References cited:
- WO-A-93/09814
- US-A- 3 813 404
- US-A- 5 374 636
- CARROLL F I ET AL: "Synthesis and ligand binding of 3.beta.-(3-substituted phenyl)- and 3.beta.-(3,4-disubstituted phenyl)tropane-2.beta.-carboxylic acid methyl esters" MED. CHEM. RES. (MCREEB,10542523);1991; VOL.1 (6); PP.382-7, XP002116826 Res. Triangle Inst.;Chem. Life Sci.; Research Triangle Park; 27709; NC; USA (US)
- CARROLL F I ET AL: "Cocaine receptor: biochemical characterization and structure-activity relationships of cocaine analogs at the dopamine transporter" J. MED. CHEM. (JMCMAR,00222623);1992; VOL.35 (6); PP.969-81, XP002116827 Research Triangle Inst.;Research Triangle Park; 27709; NC; USA (US)
- CARROLL F I ET AL: "Cocaine receptor-design of ligands" NIDA RES. MONOGR. (MIDAD4,03618595);1990; VOL.96 (DRUGS ABUSE: CHEM., PHARMACOL., IMMUNOL., AIDS); PP.112-21, XP002116828 Research Triangle Inst.;Research Triangle Park; 27709; NC; USA (US)
- CARROLL F I ET AL: "3-Aryl-2-(3'-substituted-1',2',4'-oxadiaz ol-5'-yl)tropane analogs of cocaine: affinities at the cocaine binding site at the dopamine, serotonin, and norepinephrine transporters" J. MED. CHEM. (JMCMAR,00222623);1993; VOL.36 (20); PP.2886-90, XP002116829 Research Triangle Inst.;Research Triangle Park; 27709; NC; USA (US)
- BIOCHEMICAL PHARMACOLOGY, 1986, Vol. 35, No. 7, REITH et al., "Structural Requirement for Cocaine Congeners to Interact with Dopamine and Serotonin Uptake Sites in Mouse Brain and to Induce Stereotyped Behavior", pages 1123-1129.

## Description

This application is a continuation-in-part application of U.S. Patent Application 08/506,541, filed July 24, 1995, which is a continuation-in-part of (1) U.S. Patent Application 07/972,472, filed March 23, 1993, which issued May 9, 1995 as U.S. Patent 5,413,779; (2) U.S. Patent Application 08/164,576, filed December 10, 1993, which is in turn a continuation-in-part of U.S. Patent Application 07/792,648, filed November 15, 1991, now U.S. Patent No. 5,380,848, which is in turn a continuation-in-part of U.S. Patent Application 07/564,755, filed August 9, 1990, now U.S. Patent 5,128,118 and U.S. PCT Application PCT/US91/05553, filed August 9, 1991, filed in the U.S. PCT Receiving Office and designating the United States; and (3) U.S. Patent Application 08/436,970, filed May 8, 1995, all of which are incorporated herein by reference in their entirety.

### Field of the Invention:

This invention is directed to a class of binding ligands for cocaine receptors and other receptors in the brain. Specifically, a novel family of compounds shows high binding specificity and activity, and, in a radiolabeled form, can be used to bind to these receptors, for biochemical assays and imaging techniques. Such imaging is useful for determining effective doses of new drug candidates in human populations. In addition, the higl specificity, slow onset and long duration of the action of these compounds at the receptors makes them particularly well suited for therapeutic uses, for example as substitute medication for psychostimulant abuse. Some of these compounds may be useful in treating Parkinson's Disease or depression, by virtue of their inhibitory properties at monoamine transporters.

### DISCLOSURE OF PARENT APPLICATIONS:

This application claims priority, *inter alia*, from of U.S. Patent Application 07/972,472 filed March 23, 1993, now U.S. Patent 5,413,779, the entirety of which is incorporated by reference. This application also claims priority from U.S. Patent Application 07/564,755, now U.S. Patent 5,128,118, and U.S. PCT Application PCT/US91/05553 (the U.S. National Phase of which is U.S. 07/972,472), filed August 9, 1991, both applications being incorporated herein by reference. In U.S. Application Serial No. 07/564,755, there is disclosure of a family of compounds exhibiting particularly high specificity and affinity for cocaine receptors and other . neurotransmitter receptors in the brain of the formula:

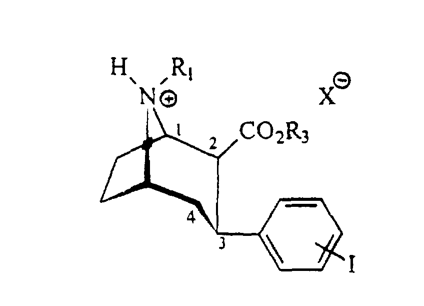

Where the broken line represents an optional chemical bond and the substituents at 2 and 3 may be at any position;

The iodo substituent may be at o, m, p, or multisubstituted;
R₁ = CH₃, CH₂CH=CH₂, (CH₂)ₙC₆H₅ n = 1-4;
R₂ = CH₃, C₂H₅, CH₃(CH₂)₃, (CH₃)₂CH, C₆H₅, C₆H₅CH₂, C₆H₅(CH₂)₂;
X = pharmacologically acceptable anion
Sites of specific interest included cocaine receptors associated with dopamine (DA) transporter sites.

Subsequently, in the U.S. PCT Application from which priority is claimed, and which is incorporated herein by reference, the values for R₁ and R₂ were expanded, such that R₁ may be an alkyl of 1-7 carbon atoms, CH₂CR₃=CR₄R₅ wherein R₃-R₅ are each, independently C₁₋₆ alkyl, or phenyl compounds of the formula C₆H₅(CH₂)_{y}, wherein y = 1-6. The PCT filing also reveals the affinity of these compounds for cocaine receptors associated with serotonin (5-hydroxytryptamine, 5-HT) transporters, and confirms, for the first time, that the *in vitro* binding reported in the earlier-filed application, is confirmed in *in vivo* testing. Specific disclosure for a variety of applications, including using the compounds in both PET and SPECT scanning, wherein either the iodine substituent, or one of the carbon groups is radioactive (I-123, 125 or 131 and C11) thus providing methods for scanning for the presence of specific cocaine receptors. Such scanning processes may be used to determine physiological conditions associated with dopamine and serotonin reuptabe inhibitors, which lead to behavioral and neurodegenerative disorders/diseases. Such disorders include depression, bipolar disorder, eating disorders, obesity, attention deficit disorder, panic attacks and disorders, obsessive-compulsive disorder, Parkinson's Disease, and cocaine, nicotine and alcohol addiction. These compounds, in addition to being used in treatment of these disorders, may be used to examine in general the density and distribution of specific cocaine receptors in various parts of the brain and/or body, to determine the efficacy of neurological treatments aimed at halting or reversing the degeneration of specific nerves in the brain, and for screening drugs, such as antidepressant drugs.

The affinity and specificity of these compounds, as reported in the applications incorporated, is surprisingly high, and compared with prior art compounds, such as [³H]WIN 35,428, the novel compounds of these applications exhibit extremely low IC₅₀ values for binding inhibition.

In U.S. Patent Application 08/164,576, filed December 10, 1993, also incorporated herein by reference in its entirety, a family of compounds was disclosed, having the formula:

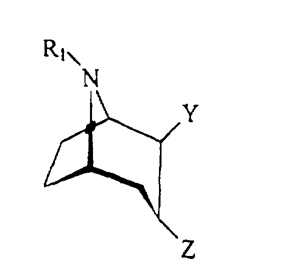

Wherein Y is 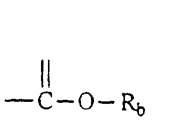 or 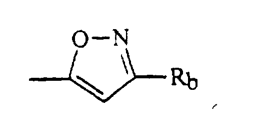 Wherein
R₁ is hydrogen, C₁₋₅ alkyl
Rₐ is phenyl, C₁₋₆ alkyl, C₁₋₆ alkyl-substituted phenyl
R_{b} is C₁₋₆ alkyl, phenyl, C₁₋₆ alkyl substituted phenyl and
Z is phenyl or naphtyl bearing 1-3 substituents selected from the group consisting of F, Cl, I, and C₁₋₆ alkyl.

These compounds exhibit unusually high affinity and specificity for binding to receptors for the dopamine transporter site, as well as the serotonin transporter site, based on inhibition of [³H]paroxetine binding. This high affinity makes certain of these compounds particularly well suited for use as therapeutic agents, as well as for imaging agents for dopamine and serotonin transporters.

### SUMMARY OF THE INVENTION

Accordingly, one object of this invention is to provide novel compounds which bind to cocaine receptors.

Another object of the invention is to provide novel 3-(substituted phenyl)-2-(substituted)tropane analogs which bind to cocaine receptors.

Still another object of the invention is to provide 3-(substituted phenyl)-2-(substituted)tropane analogs which bind preferentially to the dopamine transporter.

Yet another object of the invention is to provide 3-(substituted phenyl)-2-(substituted)tropane analogs which bind preferentially to the serotonin transporter.

Another object of the invention is to provide a compound of the formula 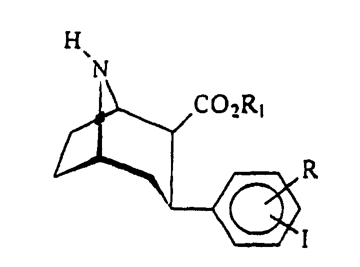 wherein R is CH₃, C₂H₅, CH₂CH₂CH₃, or CH(CH₃)₂, CH=CH₂, CH₃-C=CH₂, CH₂-CH=CH₂, CH=CHCH₃, R₁ is CH₃, CH₂C₆H₅, (CH₂)₂C₆H₅, (CH₂)₃C₆H₅, or 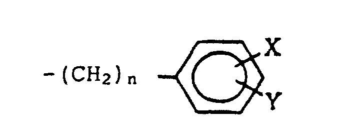 wherein X is H, OCH₃, or Cl and Y is H, OCH₃, or Cl, and n=1-8.

Another object of the invention is to provide compounds having the following formulas: 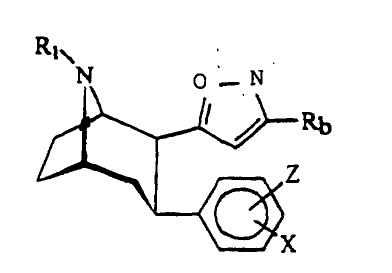 wherein
R₁=hydrogen, C₁₋₅ alkyl,
X=H, C₁₋₆ alkyl, C₃₋₈ cycloalkyl, C₁₋₄ alkoxy, C₁₋₆ alkynyl, halogen, amino, acylamido, and
Z=H, I, Br, Cl, F, CN, CF₃, NO₂, N₃, OR₁, CONH₂, CO₂R₁, C₁₋₆ alkyl, NR₄R₅, NHCOR₅, NHCO₂R₆,
R_{b} is C₁₋₆ alkyl, phenyl, C₁₋₆ alkyl substituted phenyl

A further object of the invention is to provide a pharmaceutical composition for treating psychostimulant abuse, comprising a pharmaceutically efefctive amount of a 3-(substituted phenyl)-2-(substituted) tropane analog.

A still further object of the invention is to provide a pharmaceutical composition for inhibiting the action of a psychostimulant, comprising a psychostimulant-inhibiting amount of a 3-(substituted phenyl)-2-(substituted) tropane analog.

Still another object of the invention is to provide a pharmaceutical composition for inhibiting neurotransmitter re-uptake comprising a neurotransmitter transporter-inhibiting amount of a 3-(substituted phenyl)-2-(substituted) tropane analog.

Another object of the invention is to provide a pharmaceutical composition for treating neurodegenerative disorders, comprising a pharmaceutically effective amount of a 3-(substituted phenyl) -2- (substituted) tropane analog.

Still another object of the invention is to provide a pharmaceutical composition for treating depression, comprising a pharmaceutically effective amount of a 3-(substituted phenyl)-2-(substituted) tropane analog.

Briefly, the invention pertains to the discovery that certain cocaine analogs are particularly well suited for therapeutic use as neurochemical agents. These particular cocaine analogs, in modulating neurotransmitter actions, may also be useful for modulating the actions of pyschostimulant drugs, for modulating endocrine function, for modulating motor function, and for modulating complex behaviors.

With the foregoing and other objects, advantages and features of the invention that will become here in after apparent, the nature of the invention may be more clearly understood by reference to the following detailed description of the preferred embodiments of the invention and to the appended claims.

### BRIEF DESCRIPTION OF THE DRAWINGS:

A more complete appreciation of the invention and many of the attendant advantages thereof will be readily obtained as the same becomes better understood by reference to the following detailed description when considered in connection with the accompanying drawings, wherein:
Figure 1 depicts the scheme for converting 3-(substituted phenyl)-2-tropane carboxylic acid (tropane acid) to 2-substituted tetrazoles, oxazoles. oxadiazoles, thiazoles, thiadiazoles and benzothiazole.
Figure 2 depicts the scheme in which the carboxamide obtained from the tropane acid was treated to obtain nitriles and tetrazoles.
Figure 3 depicts the scheme used to prepare 3-substituted isoxazoles.

### DETAILED DESCRIPTION OF THE INVENTION

The present invention includes novel compounds having the following formula:

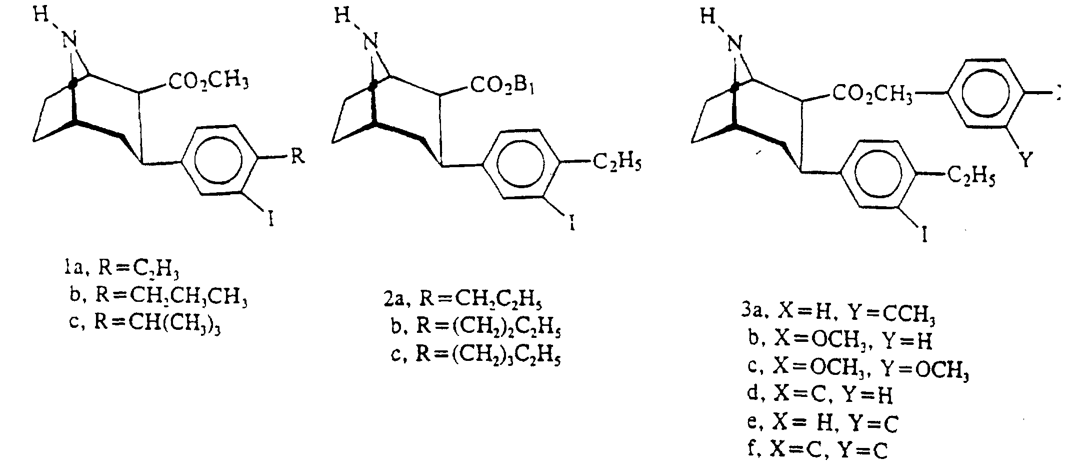

The compounds of this invention can be prepared according to the synthesis methods described in the parent applications. Alternative synthesis for related compounds will be apparent to those of ordinary skill in the art. Particular synthesis schemes are exemplified in U.S. Patent No. 5,444,070, which is incorporated herein in its entirety. Additional schemes follow hersinbelow.

### Preparation of 3β-(Substituted phenyl)tropane-2β-heterocyclic Analogues

### Chemistry

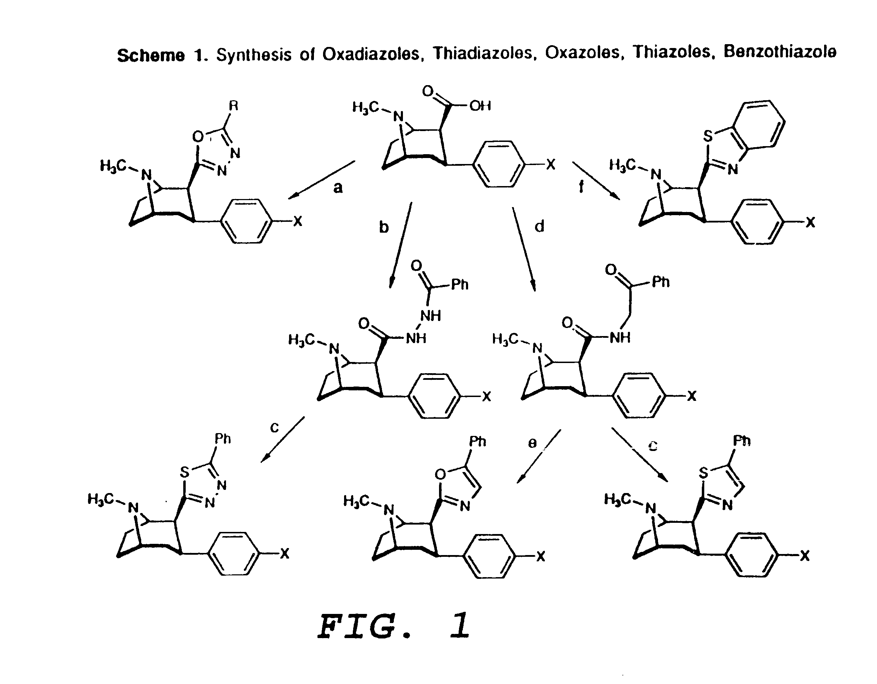

The known 2β-(substituted phenyl) -2β-tropane carboxylic acid (tropane acid) (Carroll et al., *J. Med. Chem.* **35**:1813-1817 (1992)) served as the starting material for the synthesis of 2β-substituted tetrazoles, oxazoles, oxadiazoles, thiazoles, thiadiazoles and benzothiazole as shown in Figure 1.

The tropane acid was refluxed with N-acetyl and benzoic hydrazide in phosphorous oxychloride to obtain the corresponding 5-substituted 1,3,4-oxadiazoles (Afanasiadi et al., *Chem. Heterocyclic Compd*. 397-400 (1995)). N-benzoyl hydrazide amide obtained by the reaction of the acid chloride of tropane acid with N-benzoic hydrazide was cyclized with Lawesson's reagent (El-Barbary et al., *Acta Chimica Scandinavica* 597-601 (1980)) in refluxing THF to the 5-substituted 1,3,4-thiadiazoles. The N-phenylacyl carboxamide obtained from tropane acid and 2-aminoacetophenone was cyclized by refluxing the amide in phosphorous oxychloride to obtain the required 5-substituted oxazoles (Carroll et al., *Med. Chem. Res.* 3:468 (1993)). Cyclization of the same amide with Lawesson's reagent (El-Barbary et al., 1980) in refluxing THF gave the 5-substituted thiazoles respectively. The benzothiazole was obtained without the cyclization step by the reaction of acid chloride obtained from the appropriate tropane acid with 2-aminothiophenol.

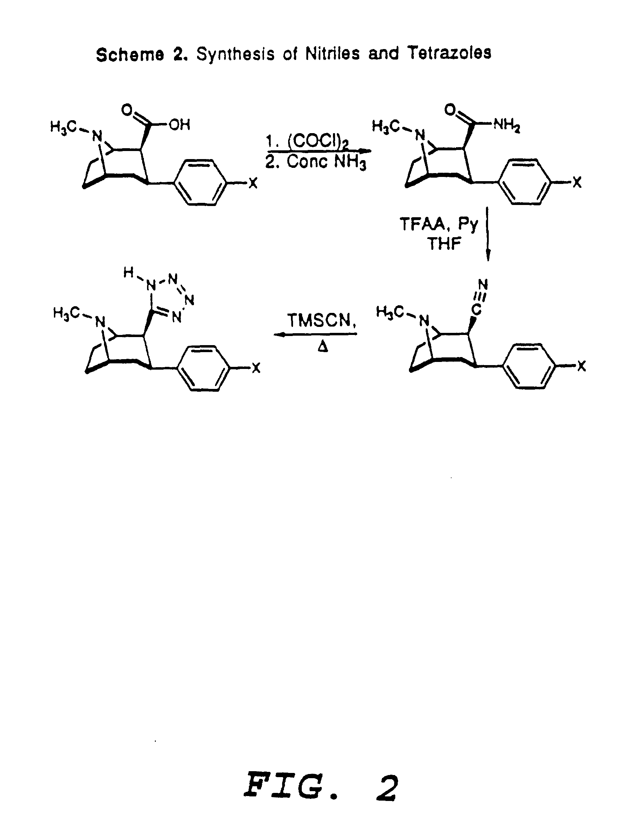

The previously reported carboxamide (Carroll et al., 1993) obtained from the tropane acid was dehydrated with trifluoroacetic acid and pyridine in THF to the nitriles (Campagna et al., *Tet. Letts.* **22**:1813-1816 (1977)) as shown in Figure 2. Cycloaddition of trimethylsilylazide to the nitrile afforded the corresponding tetrazoles (Saunders et al., *Med. Chem.* **33**:1128-1138 (1990)).

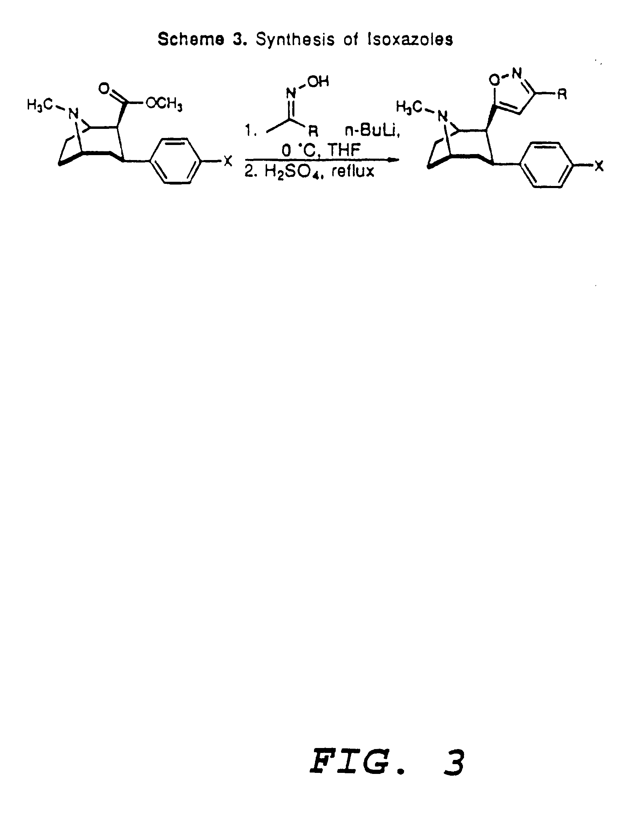

Figure 3 outlines the route used to prepare 3-substituted isoxazole. The known tropane compounds (Carroll et al., *J. Med. Chem.* **34**:2719-2725 (1991)) were treated with dilithiated methyl or phenyl acetoneoximes, obtained by the treatment of acetone or acetophenoneoxime with n-BuLi at 0°C. The corresponding addition product was cyclized without isolation using suifuric acid at reflux temperature to furnish the required isoxazoles (Saunders et al., 1990).

The therapeutic effects of the present cocaine analogs can be analyzed in various ways, many of which are well known to those of skill in the art. In particular, both *in vitro* and *in vivo* assay systems may be used for the screening of potential drugs which act as agonists or antagonists at cocaine receptors, or drugs which are effective to modulate neurotransmitter level or activity, in particular by binding to a transporter of that neurotransmitter.

The compounds of the invention may be prepared and labeled with any detectable moiety, in particular a radioactive element, and may then be introduced into a tissue or cellular sample. After the labeled material or its binding partner(s) has had an opportunity to react with sites within the sample, the location and concentration of binding of the compound may be examined by known techniques, which may vary with the nature of the label attached,

Illustrative *in* vitro assays for binding are described in Boja et al *Ann. NY Acad. Sci.* 654:282-291 (1992), which is incorporated herein by reference in its entirety. A particularly preferred *in vitro* assay involves the ability of a compound in question to displace the binding of a known labelled compound to binding sites in a tissue sample, isolated membranes or synaptosomes. Alternatively, the compounds may be analyzed by their ability to inhibit reuptake of a labelled neurotransmitter in a sample, in particular, in synaptosomes.

The compound or its binding partner(s) can also be labeled with any detectable moiety, but are preferably labelled with a radioactive element. The radioactive label can be detected by any of the currently available counting procedures, including the imaging procedures detailed in the disclosures of the parent applications. The preferred isotope may be selected from ³H, ¹¹C, ¹⁴C, ¹¹C, ¹²P, ³⁵S, ³⁶Cl, ⁵¹Cr, ⁵⁷Co, ⁵⁸Co, ⁵⁹Fe, ⁹⁰Y, ¹²⁵I ¹³¹I, and ¹⁸⁶Re.c,

As noted in the parent disclosures, the binding of the labelled compounds may be analyzed by various imaging techniques, including positron emission tomography (PET), single photon emission computed tomography (SPECT), autoradiogram, and the like. Such imaging techniques are useful for determining effective doses of new drug candidates. By performing *in vivo* competition studies, it is possible to use brain imaging studies to determine the oral doses of new drug candidates, which produce significant receptor occupancy in the brain. *In vivo* displacement studies which determine *in vivo* IC50's which in turn reflect doses that occupy receptors *in vivo* are described in Cline et al ((1992) **Synapse 12**:37-46). In addition to its uses in determining *in vivo* potency/occupancy, these same brain imaging methods can be used to determine rate of entry of compounds into the brain (Stathis et al (1995) *Psychopharmacology* **119:**376-384) and duration of action (Volkow et al (1995) *Synapse* **19:**206-211).

The binding of the compounds of the invention may be at any location where a receptor for a particular psychostimulant is present, and more specifically, any location where a dopamine or serotonin transporter is present. Such locations are in general any area comprising a part of the dopamine or serotonin pathway, in particular at synapses. Examples of locations known to be associated with dopamine transport include the cerebral cortex, hypothalamus, substantia nigra, nucleus accumbens, arcuate nucleus, anterior periventricular nuclei, median eminence and amygdala. Examples of locations known to be associated with serotonin include the striatum, cerebral cortex, hypothalamus, Raphe nuclei, pre-optic area and suprachiasmatic nucleus.

By "psychostimulant" is meant any compounds whose abuse is dependent upon mesolimbic and mesocortical dopaminergic pathways. In particular, psychostimulant relates to cocaine. However, the compounds of the invention may also be used to treat abuse of compounds not traditionally classified as "psychostimulants," but which act at a dopamine or serotonin transporter. Such abused compounds include ethanol and nicotine.

For *in vivo* studies, the compounds of the invention may be prepared in pharmaceutical compositions, with a suitable carrier and at a strength effective for administration by various means to a patient experiencing an adverse medical condition associated with cocaine receptor binding or neurotransmitter release and reuptake, for the treatment thereof. The action of the compounds may be analyzed by the imaging methods noted above, and also by behavioral studies. In particular, the pharmaceutical effects of the compounds of the invention may be reflected in locomotor activity, including the induction of ipsilateral rotation, stereotyped sniffing and the "swim test" , in schedule-controlled operant behavior (i.e., response for food or shock termination) or drug self-administration. In general, maximal behavioral effects are seen at near complete occupancy of transporter sites. Such protocols are described in Boja et al (1992), Balster et al *Drug and Alcohol Dependence* **29:**145-151 (1991), Cline et al *Pharm. Exp. Ther.* **260:**1174-1179 (1992), and Cline et al *Behavioral Pharmacology* **3**:113-116 (1992), which are hereby incorporated herein by reference in their entireties.

A variety of administrative techniques may be utilized, among them oral or parenteral techniques such as subcutaneous, intravenous, intraperitoneal, intracerebral and intracerebroventricular injections, catheterizations and the like. Average quantities of the compounds may vary in accordance with the binding properties of the compound (i.e., affinity, onset and duration of binding) and in particular should be based upon the recommendations and prescription of a qualified physician or veterinarian.

The compounds of the invention preferably have a long duration of action, which is important to facilitate dosing schedules. In rats, the present compounds have a 7-10 fold longer duration of action than cocaine (Fleckenstein et al, "Highly potent cocaine analogs cause long-lasting increases in locomotor activity," *Eur. J. Pharmacol*., in press, which is incorporated herein by reference in its entirety). In addition, the present compounds also preferably have a slow rate of entry into the brain, which is important in decreasing the potential for abuse (Stathis et al, *supra*, which is incorporated herein by reference in its entirety). The present compounds enter the brain more slowly than cocaine.

The therapeutic compositions useful in practicing the therapeutic methods of this invention may include, in admixture, a pharmaceutically acceptable excipient (carrier) and one or more of the compounds of the invention, as described herein as an active ingredient.

The preparation of therapeutic compositions which contain such neuroactive compounds as active ingredients is well understood in the art. Such compositions may be prepared for oral adminstration, or as injectables, either as liquid solutions or suspensions, however, solid forms suitable for solution in, or suspension in, liquid prior to injection can also be prepared. The preparation can also be emulsified. The active therapeutic ingredient is often mixed with excipients which are pharmaceutically acceptable and compatible with the active ingredient. Suitable excipients are, for example, water, saline, dextrose, glycerol, ethanol, or the like and combinations thereof. In addition, if desired, the composition can contain minor amounts of auxiliary substances such as wetting or emulsifying agents, and pH buffering agents which enhance the effectiveness of the active ingredient. The compounds of the invention can be formulated into the therapeutic composition as neutralized pharmaceutically acceptable salt forms.

The therapeutic compositions are conventionally administered orally, by unit dose, for example. The term "unit dose" when used in reference to a therapeutic composition of the present invention refers to physically discrete units suitable as unitary dosage for humans, each unit containing a predetermined quantity of active material calculated to produce the desired therapeutic effect in association with the required diluent; i.e., carrier, or vehicle.

The compositions are administered in a manner compatible with the dosage formulation, and in a therapeutically effeccive amount. The quantity to be administered depends on the subject to be treated, the presence of other agonists and antagonists in the subject's system, and degree of binding or inhibition of binding desired. Precise amounts of active ingredient required to be administered depend on the judgment of the practitioner and are peculiar to each individual. However, suitable dosages may range from about 0.01 to about 1000, preferably about .25 to about 500, and more preferably 10 to 50 milligrams of active ingredient per kilogram body weight of individual per day and depend on the route of administration. However, the exact dosage must be determined by factoring in rate of degradation in the stomach, absorption from the stomach, other medications administered, etc. Suitable regimes for administration and are also variable, but are typified by an initial administration followed by repeated doses at one or more hour intervals by a subsequent injection or other administration. Alternatively, continuous intravenous infusion sufficient to maintain appropriate concentrations in the blood are contemplated.

The compounds of the present invention may be administered for their activities as surrogate agonist medications for cocaine, nicotine, alcohol, amphetamine and other psychostimulant abuse. Because of their favorable binding characteristics to transporters of neurotransmitters, they may be used for inhibiting the uptake of dopamine, norepinephrine, serotonin and other monoamines. The compounds of the present invention may find use as antipsychotics, antidepressants, local anesthetics, anti-Parkinsonian agents, anti-obesity drugs, drugs useful in the treatment of bipolar disorder, eating disorders, obesity, attention deficit disorder, panic attacks and disorder, obsessive-compulsive disorder, sexual dysfunction, as anticholinergic agents and as sigma receptor drugs.

The compounds of the invention may also be useful in treating neurodegenerative disorders, in particular for treating Parkinson's Disease, but also may be useful in the treatment of cocaine, nicotine and alcohol addiction.

The preferred compounds of the present invention are derived from the series of compounds designated RTI-4229. The physical properties of some of these compounds are given in Table I.

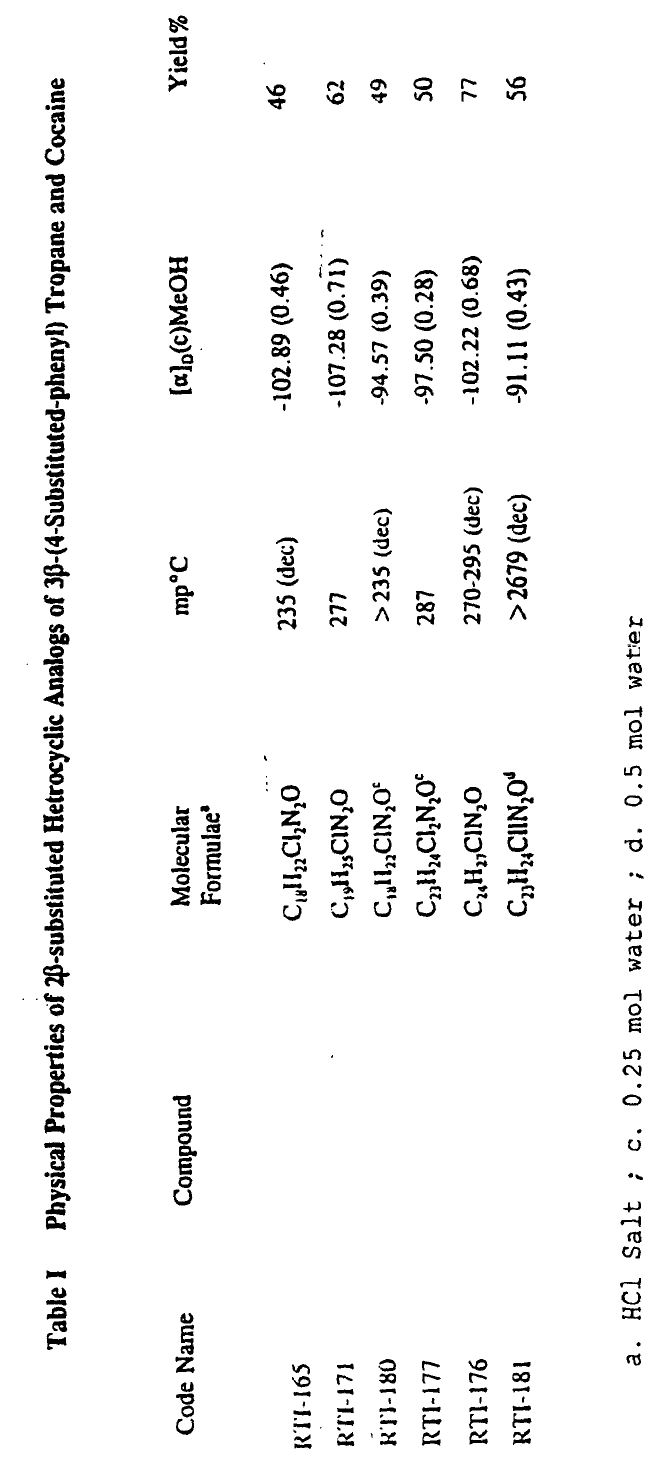

Many of the preferred compounds of the invention fall within the broad class of compounds described by the formula:

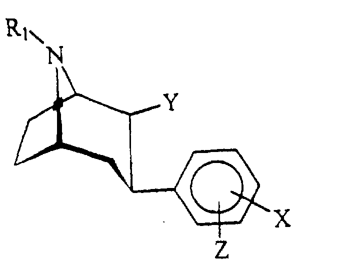

Wherein Y=CH₂R₃, CO₂R₂, CONRR¹ 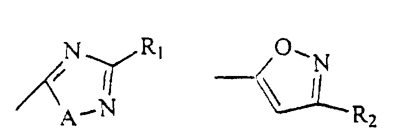
R₁=hydrogen, C₁₋₅ alkyl,
R₂=hydrogen, C₁₋₆ alkyl, C₃₋₈ cycloalkyl, C₁₋₄ alkoxy, C₁₋₆alkynyl, halogen, amine, CH₂C₆H₅, (CH₂)₂C₆H₅, (CH₂)₃C₆H₅ or 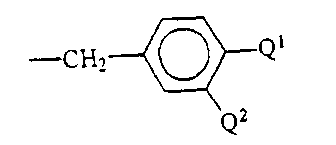
R₃=OH, hydrogen, C₁₋₆ alkyl, C₃₋₈ cycloalkyl, C₁₋₄ alkoxy, Cl, Br, I, CN, NH₂, NHC₁₋₆ alkyl, NC₁₋₆ alkyl, OCOC₁₋₆ alkyl, OCOC₁₋₃ alkylaryl,
A=S, O or N
X=H, C₁₋₆ alkyl, C₃₋₈ cycloalkyl, C₁₋₄ alkoxy, C₁₋₆ alkynyl, halogen, amino, acylamido, C₂H₅, CH₂CH₃CH₃, CH(CH₃)₂,
Z=H, I, Br, Cl, F, CN, CF₃, NO₂, N₃, OR₁, CONH₂, CO₂R₁, C₁₋₆ alkyl, NR₄R₅, NHCOR₅, NHCO₂R₆, and
Q¹ and Q² may be the same or different and = H, OCH₃, or Cl,
   wherein R₄-R₅ are each C₁₋₆ alkyl, R and R¹ are independently H, C₁₋₅ alkyl, C₁₋₆ alkene, C₁₋₆ alkyne, phenyl, phenyl substituted with 1-3 of C₁₋₆ alkyl, alkene, alkyl or alkoxy, C₁₋₆ alkoxy, phenoxy, amine, amine substituted with 1-2 of C₁₋₆ alkyl, alkene, alkyne, alkoxy or phenyl or phenoxy or R and R¹ may combine to form heterocyclic structure including pyrrolidinyl, piperidinyl and morpholino moieties, unsubstituted or substituted with 1-2 C₁₋₆ alkyl, alkene, alkyne or alkoxy groups.

The present inventors have surprisingly found that certain of the RTI-4229 series of compounds are particularly potent pharmaceutical agents in accordance with the present invention.

Preferred compounds of the RTI-4229 series include the following : RTI-4229-98, 153 and 279. The chemical structures of these compounds, along with their IC₅₀ values for inhibition of radioligand binding are given below. DA is dopamine, 5-HT is 5-hydroxytriptamine (serotonin), and NE is norepinephrine, DA=[³H]WIN 35,428; 5-HT = [³H] paroxetine and NE_{N} = [³H] nisofetine:

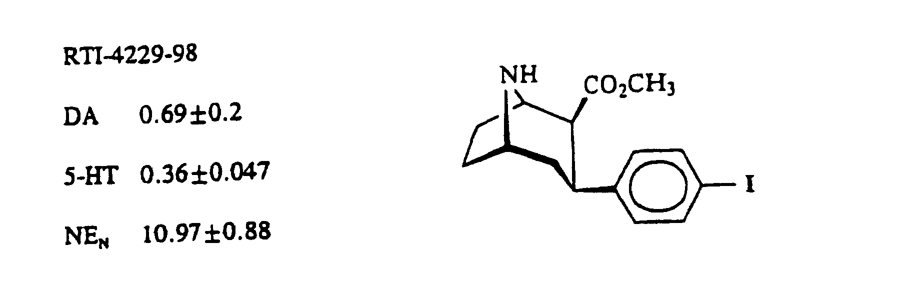

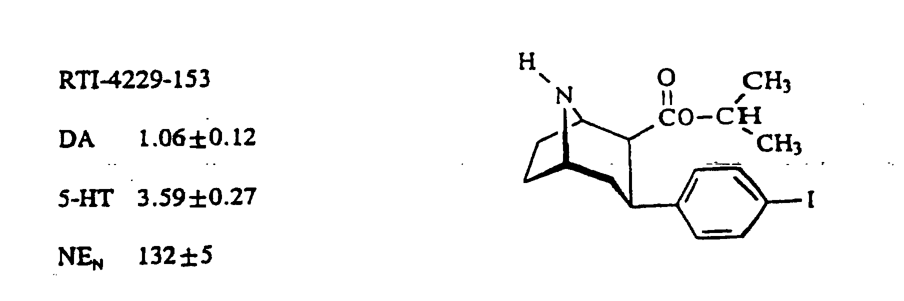

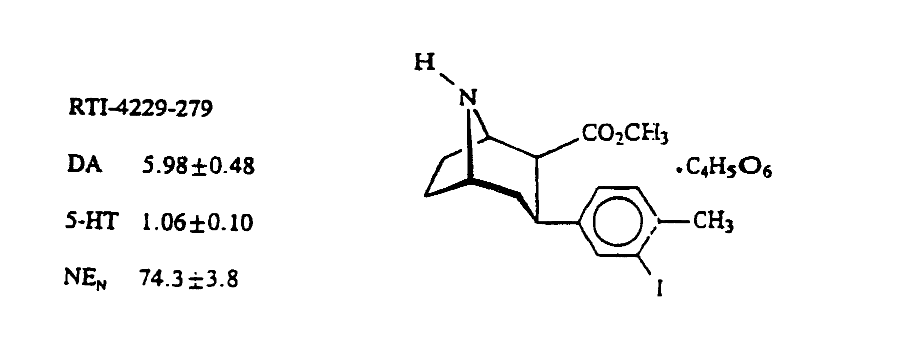

Particularly preferred compounds include RTI-4229-165, 171, 176, 177, 180, 181, 334, 335, 336, 337 and 353. The chemical structures of these compounds are given below :

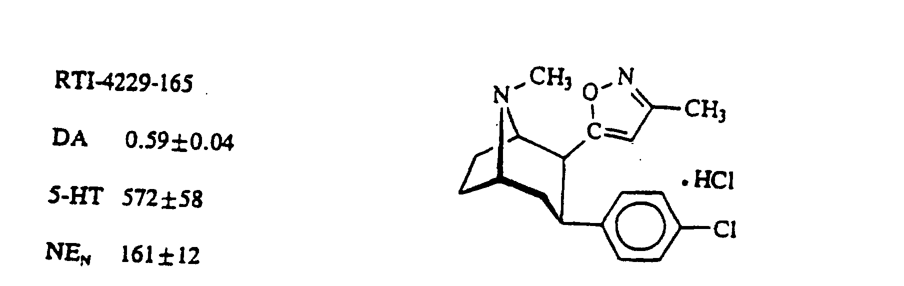

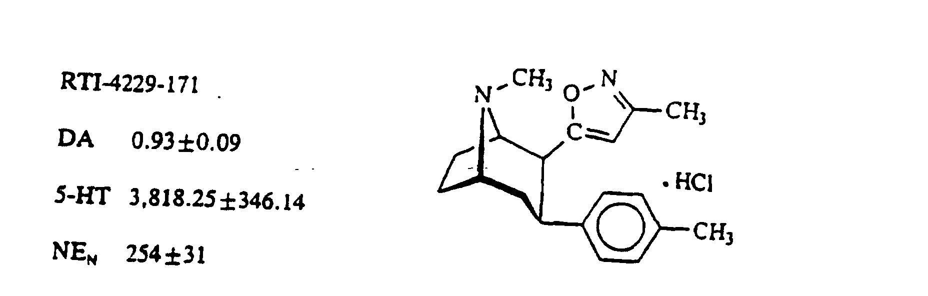

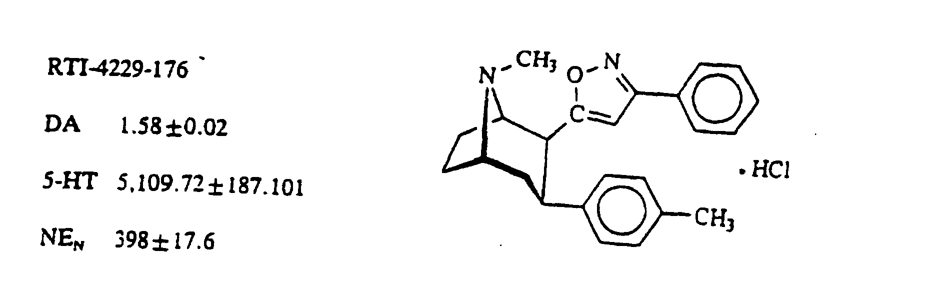

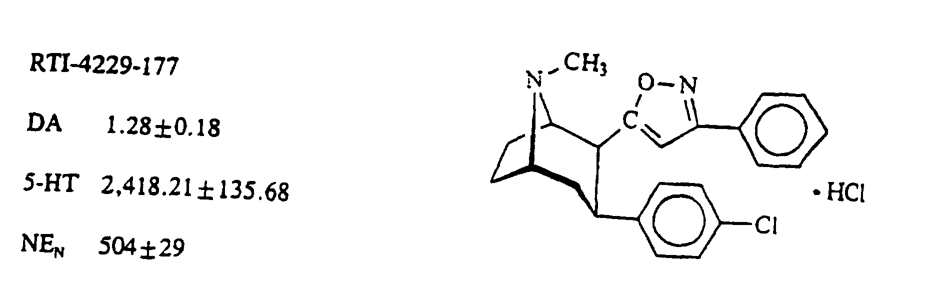

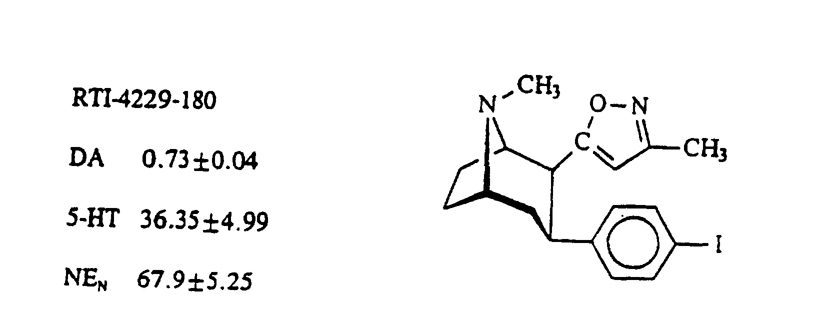

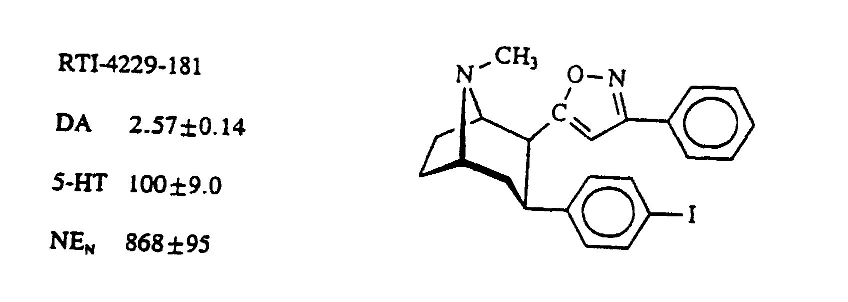

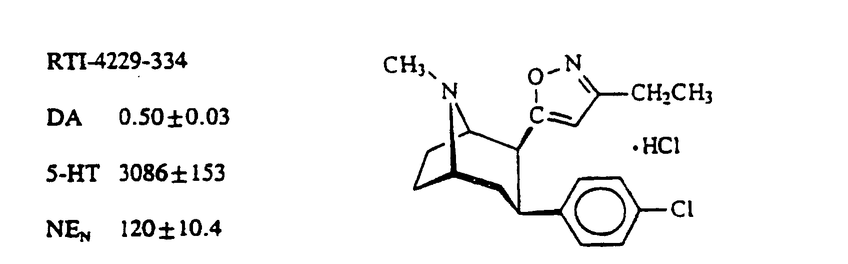

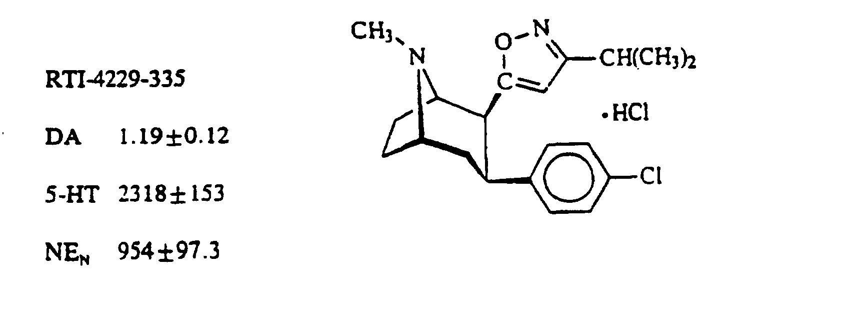

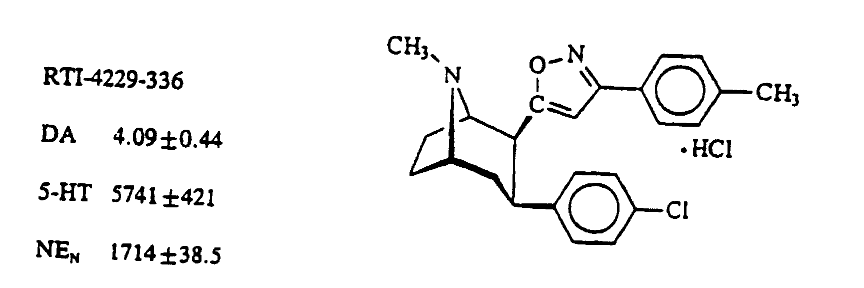

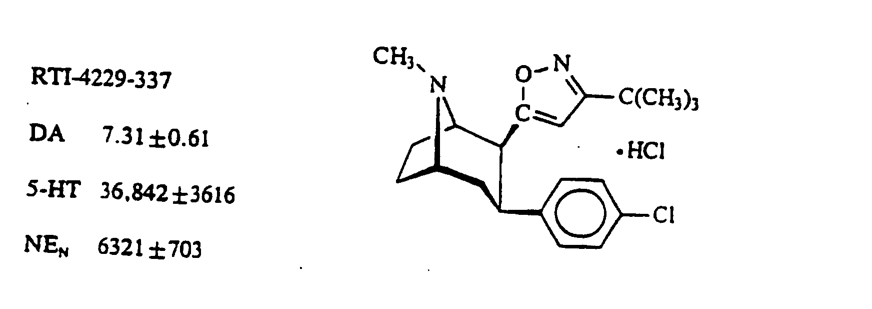

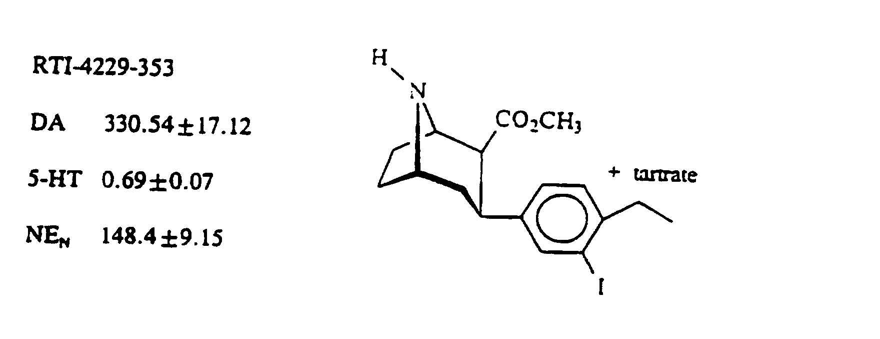

The chemical structures of the following compounds along with their IC₅₀ values for inhibition of radioligand binding are given below as a comparison.

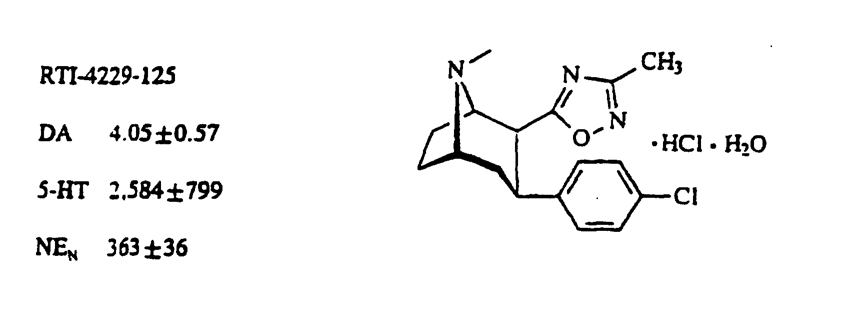

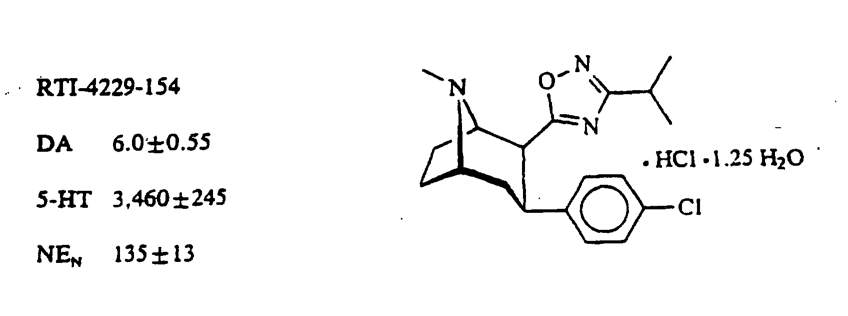

It should be noted that compound RTI-353 is a highly potent compound at the serotonin site, and is selective relative to the dopamine and norepinephrine sites. This compound is particularly useful as an antidepressant, and as an imaging agent for serotonin transporters.

Having generally described this invention, a further understanding can be obtained by reference to certain specific examples which are provided herein for purposes of illustration only and are not intended to be limiting unless otherwise specified.

### EXAMPLES

All certified grade reagents or solvents were purchased from Aldrich Chemical Co. or Fluka Chemical Co. All reagents were normally used without further purification. When anhydrous conditions were required, solvents were distilled and dried by standard techniques immediately prior to use.

All air and moisture sensitive reactions were conducted under a prepurified nitrogen atmosphere in flame-dried glassware, previously dried at 150°C. Anhydrous solvents were transferred using conventional syringe or steel canula techniques under an inert atmosphere. Removal of solvents in vacuo was done on a Buchi rotavapor rotary evaporator operated at water aspirator pressure.

¹H NMR and ¹³C NMR spectra were recorded at 250 Mhz on a Bruker AM250 spectrometer. Optical rotations were recorded on at the Sodium D line on a Rudolph Research Autopol III polarimeter (1 dm cell). Melting point was recorded on a Unimelt Thomas Hoover capillary melting point apparatus in open capillary tubes and were uncorrected. Elemental analysis were performed by Atlantic Microlab, Inc., Norcross, Georgia.

Reaction products were purified by flash column chromatography using silica gel (mesh size 230-400) purchased from VWR Scientific. Thin layer chromatography (TLC) was performed on Whatman 254 nm fluorescent silica gel 60A (1 x 3 inches, 250 [µL thickness]) precoated TLC plates using the solvent systems indicated. Developed chromatograms were evaluated under 254 nm UV light or with iodine.

### EXAMPLE 1

### General Procedure For the Preparation of Amides

To a solution of 1 mmol of 3β-(4-Chlorophenyl)-tropane-2β-carboxylic acid or 3β-(4-Methylphenyl)-tropane-2β-carboxylic acid in 5 ml of methylene chloride was added dropwise with stirring under nitrogen 2.0 eq oxalyl chloride (2 M solution in methylene chloride). The resulting solution was stirred at room temperature for an hour after evolution of gas has ceased. The solvent was removed in vacuo at room temperature and then at high vacuum to remove residual traces of oxalyl chloride. The resulting residue of acid chloride was suspended in 5 ml methylene chloride under nitrogen at 0 °C, and 2.0 eq of the amine hydrochloride containing 4.0 eq of triethylamine, or 2.5 eq of the amine free base was added. The mixture was stirred at room temperature overnight. Aqueous 3N NaOH (5 ml) was added to basify the reaction mixture, the organic layer was separated and the aqueous layer extracted with 3 x 10 ml chloroform. The combined organic layers were dried (Na₂SO₄), filtered and the solvent removed in vacuo to give crude product. The crude was purified by flash column chromatography or crystallization.

### EXAMPLE 2

### 3β-(4-Chlorophenyl)-2β-(3-methylisoxazol-5-yl)tropane Hydrochloride (RTI-165)

A solution of n-butyl lithium in hexane 5.9 ml (2.5 M. 14.6 mmol) was added to a stirred solution of acetone oxime 0.55 g (7.3 mmol) in dry THF (15 ml) at 0 °C under nitrogen. After 1 hour, a solution of 1.65 g (5.62 mmol) 3β-(4-chlorophenyl)-2β-(carbomethoxy)tropane in 10 ml dry was added dropwise with stirring at 0°C. The solution was allowed to warm to room temperature over 18 hours. The mixture was poured into a stirred solution of concentrated sulfuric acid (3.2 g) in THF (15 ml) and water (4 ml) and was heated under reflex for 1 hour. The cooled solution was made basic using saturated aqueous K₂CO₃ (10 ml) and extracted thrice with 10 ml methylene chloride. The combined organic layers were dried (Na₂SO₄), filtered and solvent removed in vacuo to give 1.8 g of crude isoxazole. Purification of the crude residue by flash column chromatography (10% CMA in methylene chloride) gave 0.74 g (46%) of pure isoxazole RTI-165 which was further purified by crystallization from methylene chloride/hexane: ¹H NMR (CDCl₃) δ 1.71 (m, 3 H), 2.10 (m, 3 H), 2.18 (s, 3 H), 2.24 (s, 3 H), 3.20 (m, 2 H), 3.32 (m, 2 H), 6.18 (s, 1 H), 6.9 (d, J = 8 Hz, 2 H), 7.14 (d, J = 8, Hz, 2 H); IR (CCl₄) 2950, 1590, 1490, 1420, 1350, 1020, 910 cm⁻¹; mp 154-156°C; Anal calcd for C₁₈H₂₁N₂OCl; C = 68.28, H = 6.68, N = 8.84, Cl = 11.19; found C = 68.22, H = 6.69, N = 8.87, Cl = 11.19; [α]_{D} - 125.58° (c = 0.43, MeOH).

The isoxazole was crystallized as the hydrochloride salt: ¹H NMR (MeOD) δ 2.04 (s, 3 H), 2.19 (m, 1 H), 2.30 (m, 1 H), 2.48 (m, 2 H), 2.60 (m, 1 H), 2.70 (m, 1 H), 2.90 (s, 3 H), 3.68 (m, 1 H), 3.81 (m, 1 H), 4.04 (m, 1 H), 4.15 (m, 1 H), 5.55 (s, 1 H), 7.04 (d, J = 8 Hz, 2 H), 7.14 (d, J = 8 Hz, 2 H); mp >235°C (dec); Anal calcd for C₁₈H₂₂Cl₂N₂O; C = 61.19, H = 6.28, N = 7.93, Cl = 20.07; found c = 60.98, H = 6.38, N = 7.91, Cl = 19.96; [α]_{D} -102.89° (c = 0.46, MeOH).

### EXAMPLE 3

### 3β-(4-Methylphenyl)-2β-(3-methylisoxazol-5-yl)tropane Hydrochloride (RTI-171)

Reaction of 1.09 g (4 mmol) of 3β-(4-Methylphenyl)-2β-(carbomethoxy)tropane as described above for RTI-165 gave after workup 1.21 g crude isoxazole. Purification of the crude by flash column chromatography (15% CMA in methylene chloride) gave 0.73 g (62%) pure isoxazole (RTI-171) : ¹H NMR (CDCl₃) δ 1.73 (m, 3 H), 2.11 (m, 3 H), 2.17 (s, 3 H), 2.23 (s, 3 H), 2.25 (s, 3 H), 3.20 (m, 2 H), 3.32 (m, 2 H), 6.13 (s, 1 H), 6.97 (m, 4 H); IR (CCl₄) 2935, 2785, 1590, 1510, 1460, 1421, 1350, 1125, 1010, 910 cm⁻¹.

The isoxazole was crystallized as the hydrochloride salt: ¹H NMR (MeOD) δ 2.01 (s, 3 H), 2.24 (s, 3 H), 2.32 (m, 2 H), 2.42 (m, 4 H), 2.81 (s, 3 H), 3.61 (m. 1 H), 3.78 (m, 1 H), 4.03 (m, 1 H), 4.15 (m, 1 H), 5.45 (s, 1 H), 6.96 (m, 4 H); mp 277°C; Anal calcd for C₁₉H₂₅CIN₂O; C = 68.55, H = 7.57, N = 8.42, Cl = 10.65; found C = 68.65, H = 7.62, N = 8.42, Cl = 10.56; [α]_{D} -107.28° (c = 0.71, MEOH).

### EXAMPLE 4

### 3β-(4-Iodophenyl)-2β-(3-methylisoxazol-5-yl)tropane Hydrochloride (RTI-180)

Reaction of 0.73 g (1.9 mmol) of 3β-(4-Iodophenyl)-2β-(carbomethoxy)tropane as described above for RTI-165 gave after workup 0.77 g of crude isoxazole. Purification of the crude by flash column chromatography (5% CMA80 in methylene chloride) gave 0.37 g (49%) of pure isoxazole RTI-180: ¹H NMR (CDCl₃) δ 1.71 (m, 3 H), 2.12 (m, 3 H), 2.18 (s, 3 H), 2.24 (s, 3 H), 3.17 (m, 2 H), 3.33 (m, 2 H), 6.18 (s, 1 H), 6.74 (m, 2 H), 7.49 (m, 2 H); IR (CHCl₃) 2940, 1600, 1485, 1450, 1420, 1355 cm⁻¹.

The isoxazole was crystallized as the hydrochloride salt: ¹H NMR (MeOD) δ 2.11 (s, 3 H), 2.50 (m, 6 H), 2.89 (s, 3 H), 3.70 (m, 1 H), 3.90 (m, 1 H), 4.14 (m, 1 H), 4.22 (m, 1 H), 5.66 (s, 1 H), 6.96 (m, 2 H), 7.56 (m, 2 H); mp >235°C (dec); Anal calcd for C₁₈H₂₂ClIN₂O·0.25H₂O C = 48.12, H = 5.05, N = 6.24, Cl = 15.79; I = 56.50; found C = 47.84, H = 5.05, N = 6.19, Cl = 15.77; I = 56.46; [α]_{D} -94.57° (c = 0.39, MeOH).

### EXAMPLE 5

### 3β-(4-Chlorophenyl)-2β-(3-phenylisoxazol-5-yl)tropane Hydrochloride (RTI-177)

Reaction of 1.18 g (4 mmol) of 3β-(4-Chlorophenyl)-2β-(carbomethoxy)tropane as described above for RTI-165 gave after work up 1.46 g of crude isoxazole. Purification of the crude by flash column chromatography [20% (ether/triethylamine 9 : 1) in hexane] gave 0.75 g (50%) of pure isoxazole RTI-177 which was further purified by crystallizing from ether/petroleum ether: ¹H NMR (CDCl₃) δ 1.74 (m, 3 H), 2.22 (m, 3 H), 2.27 (s, 3 H), 3.24 (m, 2 H), 3.36 (m, 2 H), 6.80 (s, 1 H), 6.94 (m, 2 H), 7.12 (m, 2 H), 7.40 (m, 3 H), 7.76 (m, 2 H); IR (CHCl₃) 2940, 1600, 1590, 1490, 1450, 1405, 1350 cm⁻¹.

The isoxazole was crystallized as the hydrochloride salt: ¹H NMR (MeOD) δ 2.35 (m, 6 H), 2.84 (s, 3 H), 3.73 (m, 1 H), 4.09 (m, 1 H), 4.21 (m, 1 H), 6.12 (s, 1 H), 7.14 (m, 4 H), 7.34 (m, 3 H), 7.57 (m, 2 H); mp 287°C; Anal calcd for C₂₃H₂₄Cl₂IN₂O·0.25H₂O C = 65.79, H = 5.88, N 6.67, Cl = 16.89; found C = 65.94, H = 5.79, N = 6.68, Cl = 17.00; [α]_{D} -97.5° (c = 0.28, MeOH).

### EXAMPLE 6

### 3β-(4-Methylphenyl)-2β-(3-phenylisoxazol-5-yl)tropane Hydrochloride (RTI-176)

Reaction of 1.09 g (4 mmol) of 3β-(4-Methylphenyl)-2β-(carbomethoxy)tropane as described above for RTI-165 gave after work up 1.56 g of crude isoxazole. Purification of the crude by flash column chromatography [25% (ether/triethylamine 9 : 1) in hexane] gave 1.1 g (77%) of pure isoxazole RTI-176 which was further purified by crystallizing from methylene chloride/hexane ¹H NMR (CDCl₃) δ 1.76 (m, 3 H), 2.23 (m, 3 H), 2.24 (s, 3 H), 2.27 (s, 3 H), 3.23 (m, 2 H), 3.36 (m, 2 H), 6.74 (s, 1 H), 6.93 (m, 4 H), 7.41 (m,3 H), 7.76 (m, 2 H); IR (CCl₄) 2935, 1590, 1455, 1410, 1215 cm⁻¹

The isoxazole was crystallized as the hydrochloride salt: ¹H NMR (MeOD) δ 2.08 (m, 1 H), 2.15 (s, 3 H), 2.45 (m, 5 H), 2.84 (s, 3 H), 3.68 (m, 1 H), 3.88 (m, 1 H), 4.07 (m, 1 H), 4.22 (m, 1 H), 5.97 (s, 1 H), 7.0 (m, 4 H), 7.33 (m, 3 H), 7.54 (m, 2 H); mp 270-295°C (dec); Anal calcd for C₂₄H₂₇CIN₂O; C = 72.99, H = 6.89, N = 7.10, Cl = 8.98; found C = 72.91, H = 6.91, N = 7.15, Cl = 8.98; [α]_{D} -102.22 (c = 0.68, MeOH).

### EXAMPLE 7

### 3β-(4-Iodophenyl)-2β-(3-phenylisoxazol-5-yl)tropane Hydrochloride (RTI-181)

Reaction of 0.73 g (1.9 mmol) of 3β-(4-Iodophenyl)-2β-(carbomethoxy)tropane as described above for RTI-181 gave after workup 1.46 g of crude isoxazole. Purification of the crude by flash column chromatography [20% (ether/triethylamine 9 : 1) in hexane] gave 0.5 g (56%) of pure isoxazole RTI-181 which was further purified by crystallizing from methylene chloride/hexane: ¹H NMR (CDCl₃) δ 1.72 (m, 3 H), 2.15 (m, 2 H), 2.28 (s, 3 H), 3.22 (m, 2 H), 3.35 (m, 2 H), 6.74 (m, 2 H), 6.79 (s, 1 H), 7.44 (m, 5 H), 7.75 (m, 2 H); IR (CHCl₃) 2940, 1580, 1480, 1475, 1450, 1400, 1355, 1005 cm⁻¹

The isoxazole was crystallized as the hydrochloride salt: 1 H NMR (MeOD) δ 2.54 (m, 6 H), 2.92 (s, 3 H), 3.79 (m, 1 H), 4.05 (m, 1 H), 4.19 (m, 1 H), 4.33 (m, 1 H), 6.18 (s, 1 H), 7.02 (m, 2 H), 7.43 (m, 3 H), 7.63 (m, 4 H); mp >267°C (dec); Anal calcd for C₂₃H₂₄ClIN₂O·0.5H₂O C = 53.55, H = 4.89, N = 5.43, Cl = 13.75; I = 49.21: found C = 53.75, H = 4.87, N = 5.41, Cl = 13.68; I = 48.95; [α]_{D} -91.11° (c = 0.43, MeOH).

### EXAMPLE 8

### Biochemistry of 3β-(Substituted phenyl)-2β-(heterocyclic)tropanes

Inhibition of radioligand binding data at the dopamine, serotonin, and norepinephrine transporters are listed in Table II and III.

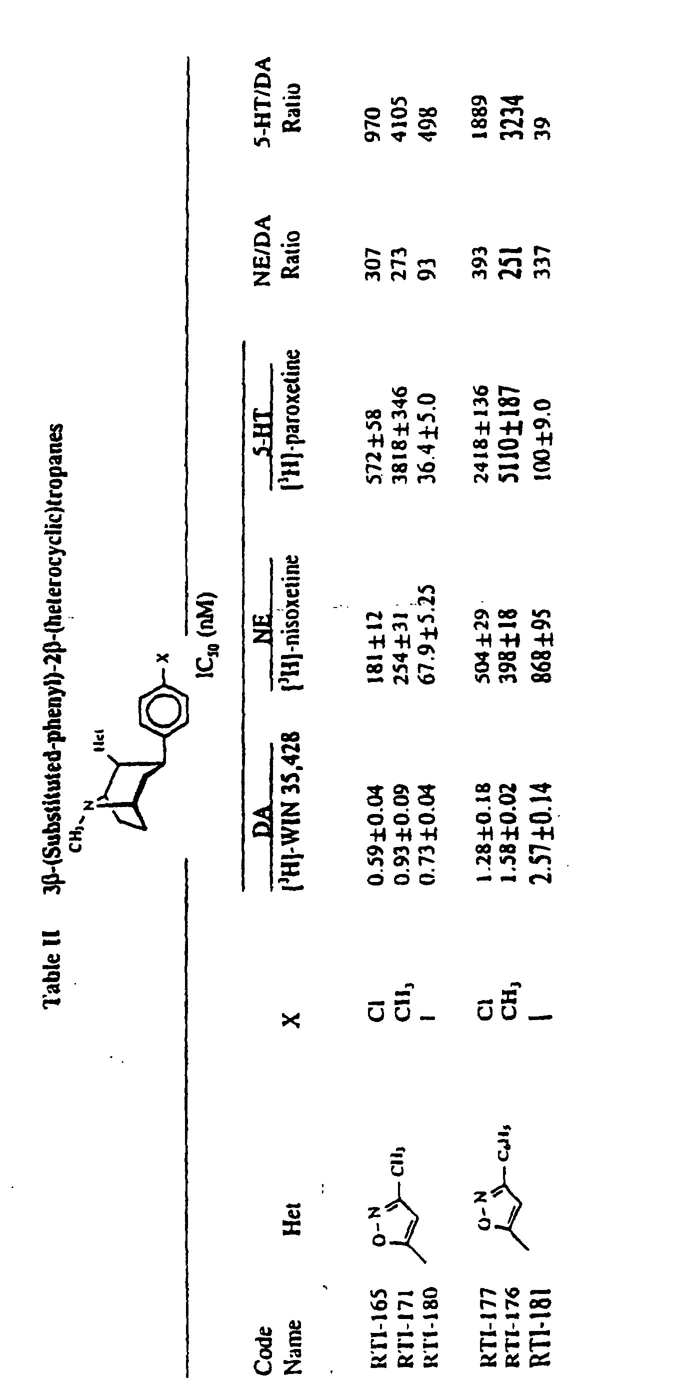

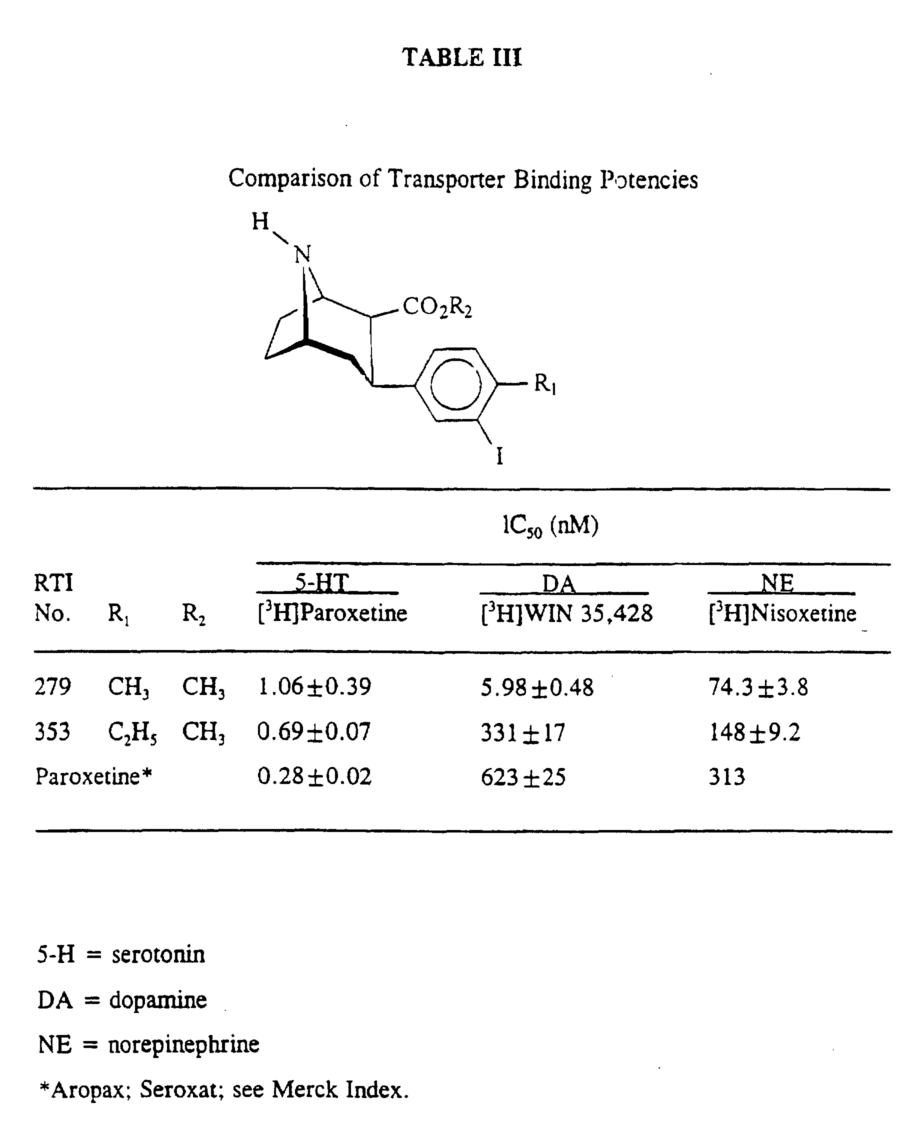

## Claims

1. A compound of the formula 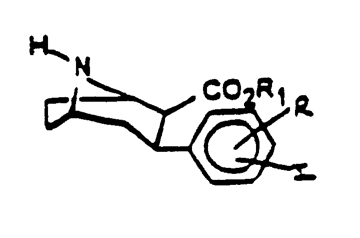 wherein R is CH₃, C₂H₅, CH₂CH₃CH₃, or CH(CH₃)₂, CH=CH₂, CH₃-C=CH₂, CH₂CH=CH₂, CH=CHCH₃, R₁ is CH₃, CH₂C₆H₅, (CH₂)₂C₆H₅, (CH₂)₃C₆H₅, or 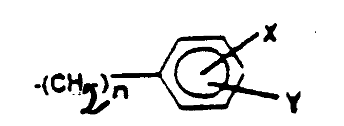 wherein X is H, OCH₃, or Cl and Y is H, OCH₃, or Cl, and n= 1-8.

2. The compound of Claim 1, wherein R₁ is CH₃.

3. The compound of Claim 2, wherein R is C₂H₅.

4. The compound of Claim 2, wherein R is CH₂CH₃CH₃.

5. The compound of Claim 2, wherein R is CH(CH₃)₂.

6. The compound of Claim 1, wherein R is C₂H₅.

7. The compound of Claim 6, wherein R₁ is CH₂C₆H₅.

8. The compound of Claim 6, wherein R₁ is (CH₂)₂C₆H₅.

9. The compound of Claim 6, wherein R₁ is (CH₂)₃C₆H₅.

10. The compound of Claim 6, wherein R₁ is

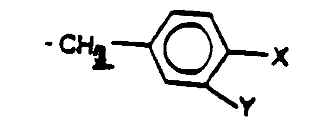

11. The compound of Claim 10, wherein X is H and Y is OCH₃.

12. The compound of Claim 10, wherein X is OCH₃ and Y is H.

13. The compound of Claim 10, wherein X is OCH₃ and Y is OCH₃.

14. The compound of Claim 10, wherein X is Cl and Y is H.

15. The compound of Claim 10, wherein X is H and Y is Cl.

16. The compound of Claim 10, wherein X is Cl and Y is Cl.

17. A pharmaceutical composition for treating psychostimulant abuse, comprising a pharmaceutically effective amount of the compound of claim 1.

18. A pharmaceutical composition for inhibiting the action of a psychostimulant, comprising a psychostimulant-inhibiting amount of the compound of claim 1.

19. A pharmaceutical composition for inhibiting neurotransmitter re-uptake, comprising a neurotransmitter transporter-inhibiting amount of the compound of claim 1.

20. A pharmaceutical composition for treating neurodegenerative disorders, comprising a pharmaceutically effective amount of the compound of claim 1.

21. A pharmaceutical composition for treating depression, comprising a pharmaceutically effective amount of the compound of claim 1.

22. A pharmaceutical composition for treating anorexia, comprising a pharmaceutically effective amount of the compound of claim 1.

23. The pharmaceutical composition of anyone of claims 17 to 21 wherein the compound has the following formula : 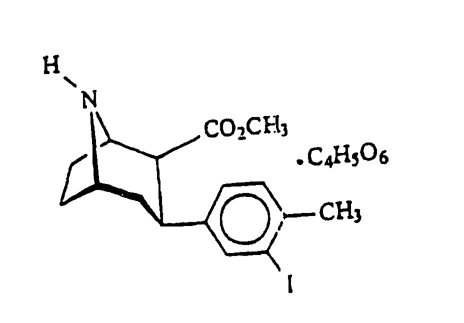 or

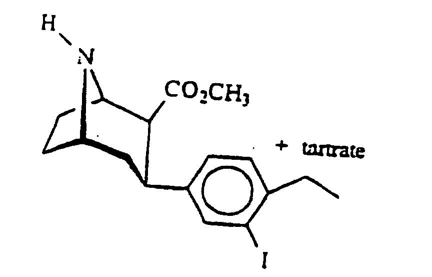

24. The composition of claim 21, wherein the compound has the following formula :

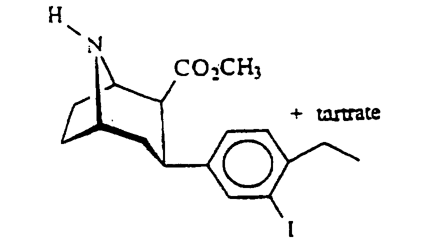

25. A compound of the formula : 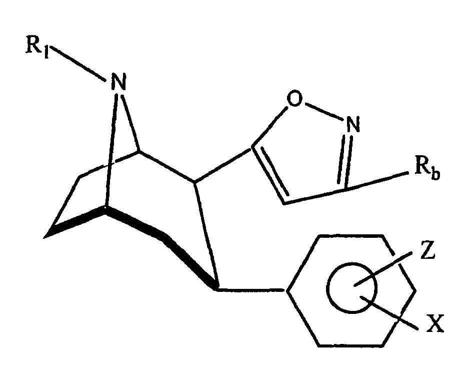 wherein
R₁=hydrogen, C₁₋₅ alkyl,
X=H, C₁₋₆ alkyl, C₃₋₈ cycloalkyl, C₁₋₄ alkoxy, C₁₋₆ alkynyl, halogen, amino or acylamido
Z=H, I, Br, Cl, F, CN, CF₃, NO₂, N₃, OR₁, CONH₂, CO₂R₁, C₁₋₆ alkyl, NR₄R₅, NHCOR₅, or NHCO₂R₆, and
R_{b} is C₁₋₆ alkyl, phenyl, or C₁₋₆ alkyl substituted phenyl.

26. A pharmaceutical composition for treating psychostimulant abuse, comprising a pharmaceutically effective amount of the compound of formula : 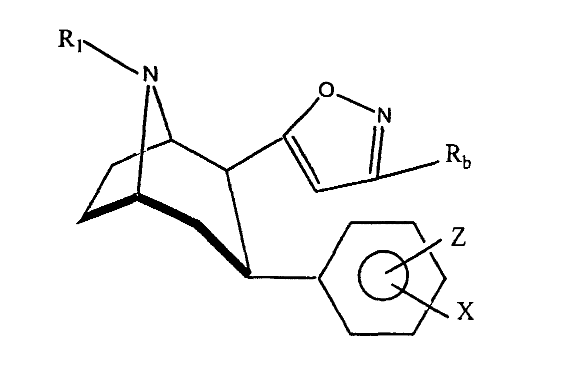 wherein
R₁=hydrogen or C₁₋₅ alkyl,
X=H, C₁₋₆ alkyl, C₃₋₈ cycloalkyl, C₁₋₄ alkoxy, C₁₋₆ alkynyl, halogen, amino or acylamido
Z=H, I, Br, Cl, F, CN, CF₃, NO₂, N₃, OR₁, CONH₂, CO₂R₁, C₁₋₆ alkyl, NR₄R₅, NHCOR₅, or NHCO₂R₆, and
R_{b} is C₁₋₆ alkyl, phenyl, or C₁₋₆ alkyl substituted phenyl.

27. A pharmaceutical composition for inhibiting the action of a psychostimulant, comprising a psychostimulant-inhibiting amount of the compound of formula : 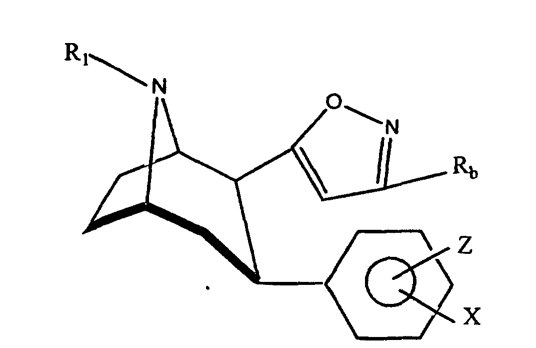 wherein
R₁=hydrogen or C₁₋₅ alkyl,
X=H, C₁₋₆ alkyl, C₃₋₈ cycloalkyl, C₁₋₄ alkoxy, C₁₋₆ alkynyl, halogen, amino or acylamido
Z=H, I, Br, Cl, F, CN, CF₃, NO₂, N₃, OR₁, CONH₂, CO₂R₁, C₁₋₆ alkyl, NR₄R₅, NHCOR₅, or NHCO₂R₆, and
R_{b} is C₁₋₆ alkyl, phenyl, or C₁₋₆ alkyl substituted phenyl.

28. A pharmaceutical composition for inhibiting neurotransmitter re-uptake, comprising a neurostransmitter transporter-inhibiting amount of the compound of formula : 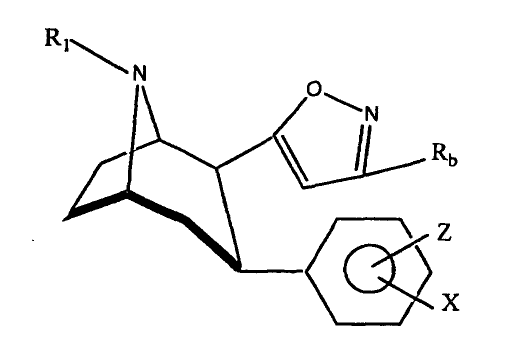 wherein
R₁=hydrogen or C₁₋₅ alkyl,
X=H, C₁₋₆ alkyl, C₃₋₈ cycloalkyl, C₁₋₄ alkoxy, C₁₋₆ alkynyl, halogen, amino or acylamido
Z=H, I, Br, Cl, F, CN, CF₃, NO₂, N₃, OR₁, CONH₂, CO₂R₁, C₁₋₆ alkyl, NR₄R₅, NHCOR₅, or NHCO₂R₆, and
R_{b} is C₁₋₆ alkyl, phenyl, or C₁₋₆ alkyl substituted phenyl.

29. A pharmaceutical composition for treating neurodegenerative disorders, comprising a pharmaceutically effective amount of the compound of formula : 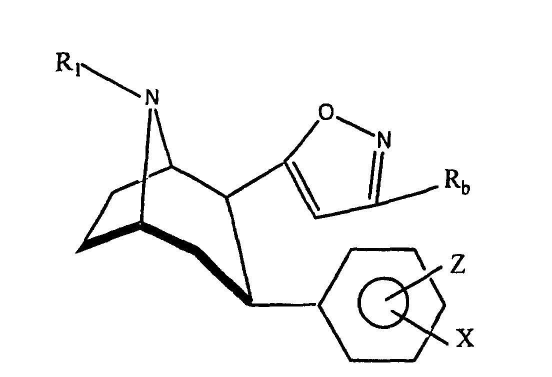 wherein
R₁=hydrogen or C₁₋₅ alkyl,
X=H, C₁₋₆ alkyl, C₃₋₈ cycloalkyl, C₁₋₄ alkoxy, C₁₋₆ alkynyl, halogen, amino or acylamido
Z=H, I, Br, Cl, F, CN, CF₃, NO₂, N₃, OR₁, CONH₂, CO₂R₁, C₁₋₆ alkyl, NR₄R₅, NHCOR₅, or NHCO₂R₆, and
R_{b} is C₁₋₆ alkyl, phenyl, or C₁₋₆ alkyl substituted phenyl.

30. A pharmaceutical composition for treating depression, comprising a pharmaceutically effective amount of the compound of formula : 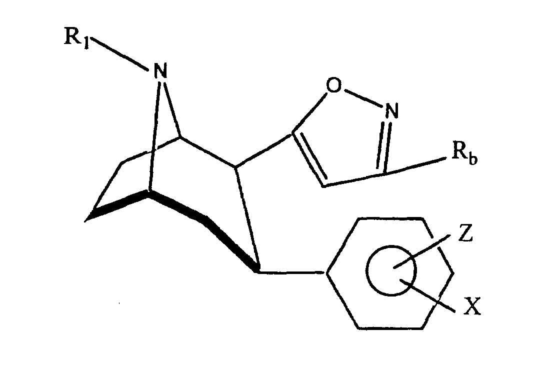 wherein
R₁=hydrogen or C₁₋₅ alkyl,
X=H, C₁₋₆ alkyl, C₃₋₈ cycloalkyl, C₁₋₄ alkoxy, C₁₋₆ alkynyl, halogen, amino or acylamido
Z=H, I, Br, Cl, F, CN, CF₃, NO₂, N₃, OR₁, CONH₂, CO₂R₁, C₁₋₆ alkyl, NR₄R₅, NHCOR₅, or NHCO₂R₆, and
R_{b} is C₁₋₆ alkyl, phenyl, or C₁₋₆ alkyl substituted phenyl.

31. A pharmaceutical composition for treating anorexia, comprising a pharmaceutically effective amount of the compound of formula : 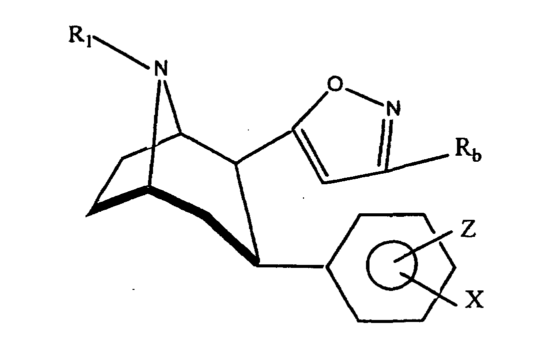 wherein
R₁=hydrogen or C₁₋₅ alkyl,
X=H, C₁₋₆ alkyl, C₃₋₈ cycloalkyl, C₁₋₄ alkoxy, C₁₋₆ alkynyl, halogen, amino or acylamido
Z=H, I, Br, Cl, F, CN, CF₃, NO₂, N₃, OR₁, CONH₂, CO₂R₁, C₁₋₆ alkyl , NR₄R₅, NHCOR₅, or NHCO₂R₆, and
R_{b} is C₁₋₆ alkyl, phenyl, or C₁₋₆ alkyl substituted phenyl.

## Patentansprüche

1. Eine Verbindung der Formel 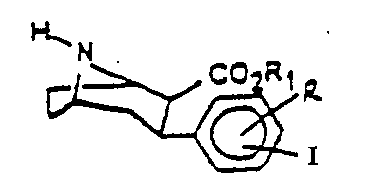 wobei R CH₃, C₂H₅, CH₂CH₃CH₃ oder CH(CH₃)₂, CH=CH₂, CH₃-C=CH₂, CH₂CH=CH₂, CH=CHCH₃ ist, R₁ CH₃, CH₂C₆H₅, (CH₂)₂C₆H₅, (CH₂)₃C₆H₅ oder 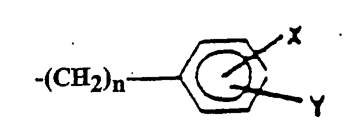 ist, wobei X H, OCH₃ oder Cl ist und Y H, OCH₃ oder Cl ist und n=1-8.

2. Die Verbindung nach Anspruch 1, wobei R₁ CH₃ ist.

3. Die Verbindung nach Anspruch 2, wobei R C₂H₅ ist.

4. Die Verbindung nach Anspruch 2, wobei R CH₂CH₃CH₃ ist.

5. Die Verbindung nach Anspruch 2, wobei R CH(CH₃)₂ ist.

6. Die Verbindung nach Anspruch 1, wobei R C₂H₅ ist.

7. Die Verbindung nach Anspruch 6, wobei R₁ CH₂C₆H₅ ist.

8. Die Verbindung nach Anspruch 6, wobei R₁ (CH₂)₂C₆H₅ ist.

9. Die Verbindung nach Anspruch 6, wobei R₁ (CH₂)₃C₆H₅ ist.

10. Die Verbindung nach Anspruch 6, wobei R₁ 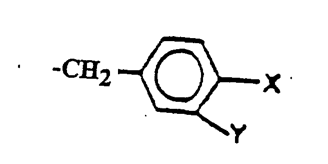 ist.

11. Die Verbindung nach Anspruch 10, wobei X H ist und Y OCH₃ ist.

12. Die Verbindung nach Anspruch 10, wobei X OCH₃ ist und Y H ist.

13. Die Verbindung nach Anspruch 10, wobei X OCH₃ ist und Y OCH₃ ist.

14. Die Verbindung nach Anspruch 10, wobei X Cl ist und Y H ist.

15. Die Verbindung nach Anspruch 10, wobei X H ist und Y Cl ist.

16. Die Verbindung nach Anspruch 10, wobei X Cl ist und Y Cl ist.

17. Eine pharmazeutische Zusammensetzung zur Behandlung von einem Mißbrauch von Psychostimulantien, die eine pharmazeutisch effektive Menge der Verbindung nach Anspruch 1 umfaßt.

18. Eine pharmazeutische Zusammensetzung zum Inhibieren der Aktivität einer Psychostimulans, die eine Psychostimulans-inhibierende Menge der Verbindung nach Anspruch 1 umfaßt.

19. Eine pharmazeutische Zusammensetzung zum Inhibieren der Wiederaufnahme von Neurotransmittern, die eine Neurotransmittertransporter-inhibierende Menge der Verbindung nach Anspruch 1 umfaßt.

20. Eine pharmazeutische Zusammensetzung zur Behandlung von neurodegenerativen Krankheiten, die eine pharmazeutisch effekte Menge der Verbindung nach Anspruch 1 umfaßt.

21. Eine pharmazeutische Zusammensetzung zur Behandlung von Depression, die eine pharmazeutisch effektive Menge der Verbindung nach Anspruch 1 umfaßt.

22. Eine pharmazeutische Zusammensetzung zur Behandlung von Anorexia, die eine pharmazeutisch effektive Menge der Verbindung nach Anspruch 1 umfaßt.

23. Die pharmazeutische Zusammensetzung von einem der Ansprüche 17 bis 21, wobei die Verbindung die folgende Formel hat: 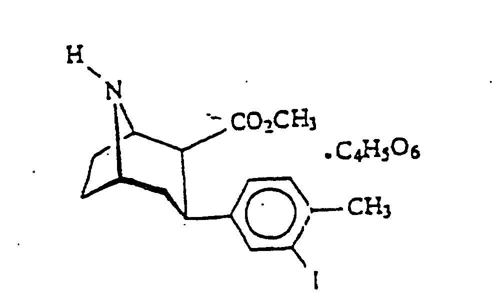 oder

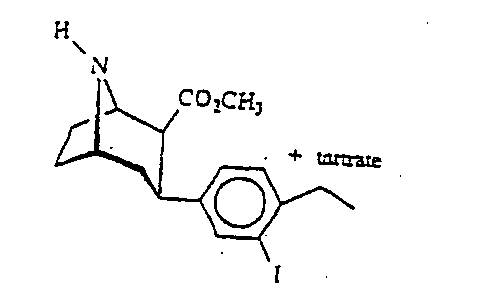

24. Die Zusammensetzung nach Anspruch 21, wobei die Verbindung die folgende Formel hat:

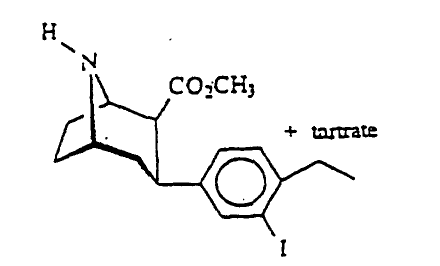

25. Eine Verbindung der Formel 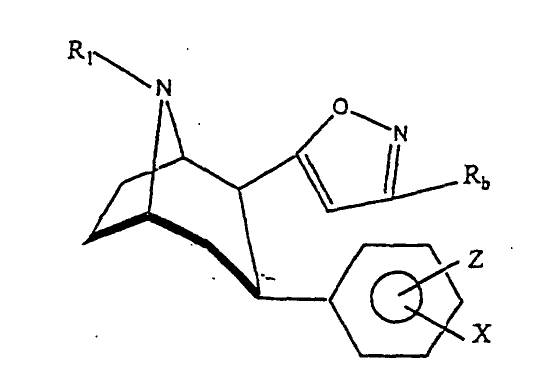 wobei
R₁=Wasserstoff, C₁₋₅-Alkyl,
X=H, C₁₋₆-Alkyl, C₃₋₈-Cycloalkyl, C₁₋₄-Alkoxy, C₁₋₆-Alkynyl, Halogen, Amino oder Acylamido
Z=H, I, Br, Cl, F, CN, CF₃, NO₂, N₃, OR₁, CONH₂, CO₂R₁, C₁₋₆-Alkyl, NR₄R₅, NHCOR₅ oder NHCO₂R₆ und
R_{b} C₁₋₆-Alkyl, Phenyl oder C₁₋₆-Alkyl-substituiertes Phenyl ist.

26. Eine pharmazeutische Zusammensetzung zur Behandlung von einem Mißbrauch von Psychostimulantien, die eine pharmazeutisch effektive Menge der Verbindung der Formel 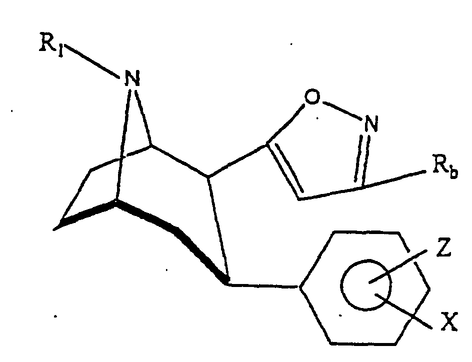 umfaßt, wobei
R₁=Wasserstoff oder C₁₋₅-Alkyl,
X=H, C₁₋₆-Alkyl, C₃₋₈-Cycloalkyl, C₁₋₄-Alkoxy, C₁₋₆-Alkynyl, Halogen, Amino oder Acylamido
Z=H, I, Br, Cl, F, CN, CF₃, NO₂, N₃, OR₁, CONH₂, CO₂R₁, C₁₋₆-Alkyl, NR₄R₅, NHCOR₅ oder NHCO₂R₆ und
R_{b} C₁₋₆-Alkyl, Phenyl oder C₁₋₆-Alkyl-substituiertes Phenyl ist.

27. Eine pharmazeutische Zusammensetzung zum Inhibieren der Aktivität einer Psychostimulans, die eine Psychostimulans-inhibierende Menge der Verbindung der Formel 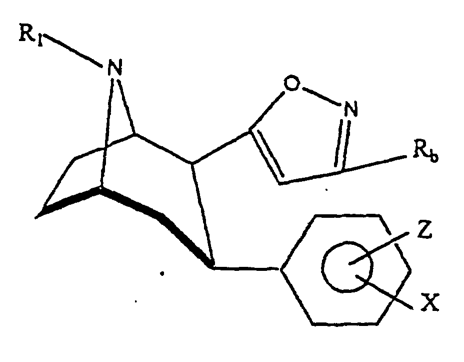 umfaßt, wobei
R₁=Wasserstoff oder C₁₋₅-Alkyl,
X=H, C₁₋₆-Alkyl, C₃₋₈-Cycloalkyl, C₁₋₄-Alkoxy, C₁₋₆-Alkynyl, Halogen, Amino oder Acylamido
Z=H, I, Br, Cl, F, CN, CF₃, NO₂, N₃, OR₁, CONH₂, CO₂R₁, C₁₋₆-Alkyl, NR₄R₅, NHCOR₅ oder NHCO₂R₆ und
R_{b} C₁₋₆-Alkyl, Phenyl oder C₁₋₆-Alkyl-substituiertes Phenyl ist.

28. Eine pharmazeutische Zusammensetzung zum Inhibieren der Wiederaufnahme von Neurotransmittern, die eine Neurotransmittertransporter-inhibierende Menge der Verbindung der Formel 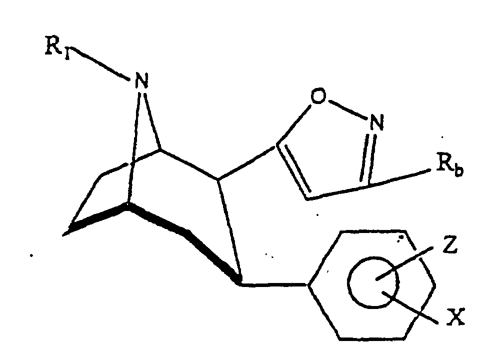 umfaßt, wobei
R₁=Wasserstoff oder C₁₋₅-Alkyl,
X=H, C₁₋₆-Alkyl, C₃₋₈-Cycloalkyl, C₁₋₄-Alkoxy, C₁₋₆-Alkynyl, Halogen, Amino oder Acylamido
Z=H, I, Br, Cl, F, CN, CF₃, NO₂, N₃, OR₁, CONH₂, CO₂R₁, C₁₋₆-Alkyl, NR₄R₅, NHCOR₅ oder NHCO₂R₆ und
R_{b} C₁₋₆-Alkyl, Phenyl oder C₁₋₆-Alkyl-substituiertes Phenyl ist.

29. Eine pharmazeutische Zusammensetzung zur Behandlung von neurodegenerativen Krankheiten, die eine pharmazeutisch effektive Menge der Verbindung der Formel 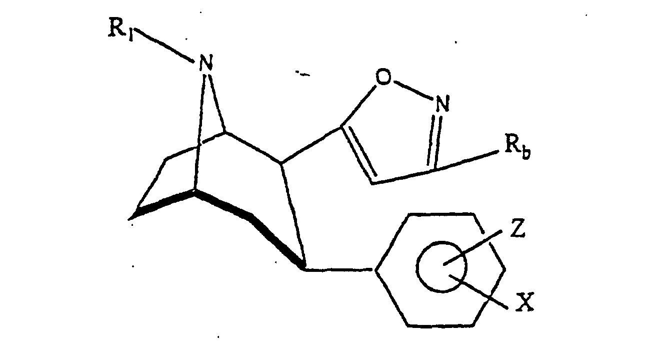 umfaßt, wobei
R₁=Wasserstoff oder C₁₋₅-Alkyl,
X=H, C₁₋₆-Alkyl, C₃₋₈-Cycloalkyl, C₁₋₄-Alkoxy, C₁₋₆-Alkynyl, Halogen, Amino oder Acylamido
Z=H, I, Br, Cl, F, CN, CF₃, NO₂, N₃, OR₁, CONH₂, CO₂R₁, C₁₋₆-Alkyl, NR₄R₅, NHCOR₅ oder NHCO₂R₆ und
R_{b} C₁₋₆-Alkyl, Phenyl oder C₁₋₆-Alkyl-substituiertes Phenyl ist.

30. Eine pharmazeutische Zusammensetzung zur Behandlung von Depression, die eine pharmazeutisch effektive Menge der Verbindung der Formel 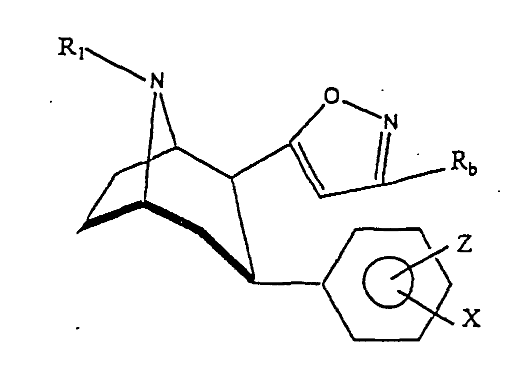 umfaßt, wobei
R₁=Wasserstoff oder C₁₋₅-Alkyl,
X=H, C₁₋₆-Alkyl, C₃₋₈-Cycloalkyl, C₁₋₄-Alkoxy, C₁₋₆-Alkynyl, Halogen, Amino oder Acylamido
Z=H, I, Br, Cl, F, CN, CF₃, NO₂, N₃, OR₁, CONH₂, CO₂R₁, C₁₋₆-Alkyl, NR₄R₅, NHCOR₅ oder NHCO₂R₆ und
R_{b} C₁₋₆-Alkyl, Phenyl oder C₁₋₆-Alkyl-substituiertes Phenyl ist.

31. Eine pharmazeutische Zusammensetzung zur Behandlung von Anorexia, die eine pharmazeutisch effektive Menge der Verbindung der Formel 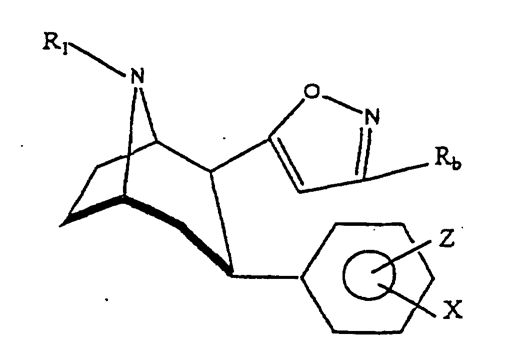 umfaßt, wobei
R₁=Wasserstoff oder C₁₋₅-Alkyl,
X=H, C₁₋₆-Alkyl, C₃₋₈-Cycloalkyl, C₁₋₄-Alkoxy, C₁₋₆-Alkynyl, Halogen, Amino oder Acylamido
Z=H, I, Br, Cl, F, CN, CF₃, NO₂, N₃, OR₁, CONH₂, CO₂R₁, C₁₋₆-Alkyl, NR₄R₅, NHCOR₅ oder NHCO₂R₆ und
R_{b} C₁₋₆-Alkyl, Phenyl oder C₁₋₆-Alkyl-substituiertes Phenyl ist.

## Revendications

1. Composé de formule : 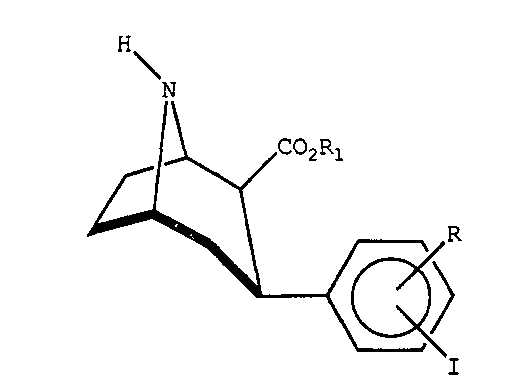 dans laquelle R est CH₃, C₂H₅, CH₂CH₃CH₃, ou CH(CH₃)₂, CH=CH₂, CH₃-C=CH₂, CH₂CH=CH₂, CH=CHCH₃, R₁ est CH₃, CH₂C₆H₅, (CH₂)₂C₆H₅, (CH₂)₃C₆H₅, ou 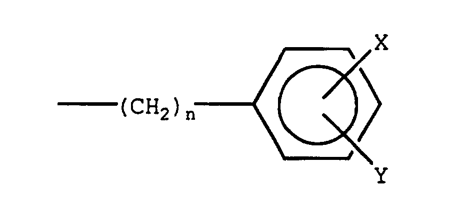 où X est H, OCH₃, ou Cl et Y est H, OCH₃, ou Cl et n=1-8.

2. Composé selon la revendication 1, dans lequel R₁ est CH₃.

3. Composé selon la revendication 2, dans lequel R est C₂H₅.

4. Composé selon la revendication 2, dans lequel R est CH₂CH₃CH₃.

5. Composé selon la revendication 2, dans lequel R est CH(CH₃)₂.

6. Composé selon la revendication 1, dans lequel R est C₂H₅.

7. Composé selon la revendication 6, dans lequel R₁ est CH₂C₆H₅.

8. Composé selon la revendication 6, dans lequel R₁ est (CH₂)₂C₆H₅.

9. Composé selon la revendication 6, dans lequel R₁ est (CH₂)₃C₆H₅.

10. Composé selon la revendication 6, dans lequel R₁ est

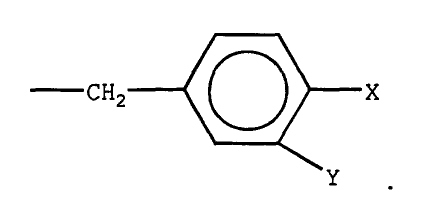

11. Composé selon la revendication 10, dans lequel X est H et Y est OCH₃.

12. Composé selon la revendication 10, dans lequel X est OCH₃ et Y est H.

13. Composé selon la revendication 10, dans lequel X est OCH₃ et Y est OCH₃.

14. Composé selon la revendication 10, dans lequel X est Cl et Y est H.

15. Composé selon la revendication 10, dans lequel X est H et Y est Cl.

16. Composé selon la revendication 10, dans lequel X est Cl et Y est Cl.

17. Composition pharmaceutique pour traiter l'abus de psychostimulants, contenant une quantité pharmaceutiquement efficace du composé selon la revendication 1.

18. Composition pharmaceutique pour inhiber l'action d'un psychostimulant, contenant une quantité du composé selon la revendication 1 apte à inhiber les psychostimulants.

19. Composition pharmaceutique pour inhiber le recaptage de neurotransmetteurs, contenant une quantité du composé selon la revendication 1 apte à inhiber les transporteurs des neurotransmetteurs.

20. Composition pharmaceutique pour traiter les troubles neurodégénératifs, contenant une quantité pharmaceutiquement efficace du composé selon la revendication 1.

21. Composition pharmaceutique pour traiter la dépression, contenant une quantité pharmaceutiquement efficace du composé selon la revendication 1.

22. Composition pharmaceutique pour traiter l'anorexie, contenant une quantité pharmaceutiquement efficace du composé selon la revendication 1.

23. Composition pharmaceutique selon l'une quelconque des revendications 17 à 21, dans laquelle le composé a la formule suivante : 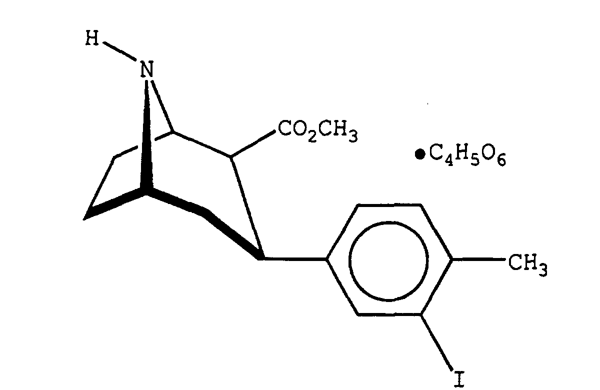 ou

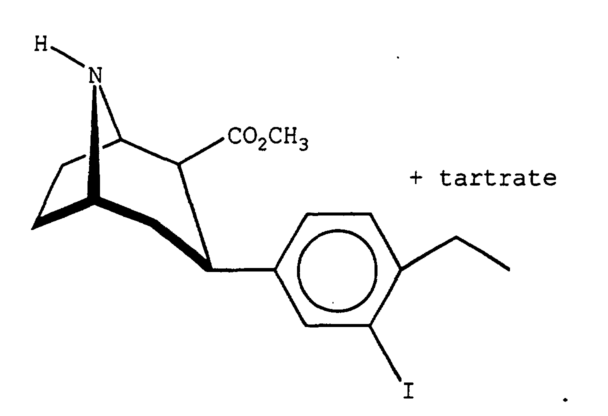

24. Composition selon la revendication 21, dans laquelle le composé a la formule suivante :

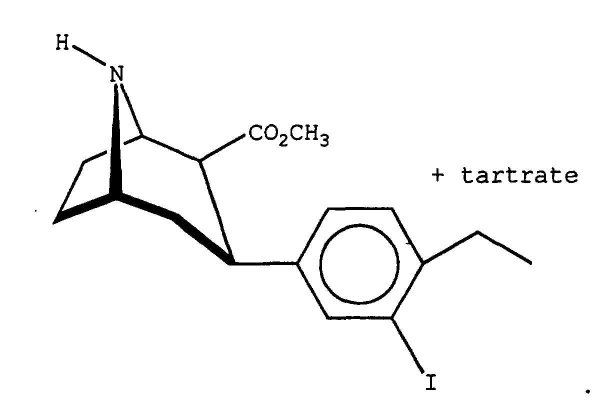

25. Composé de formule : 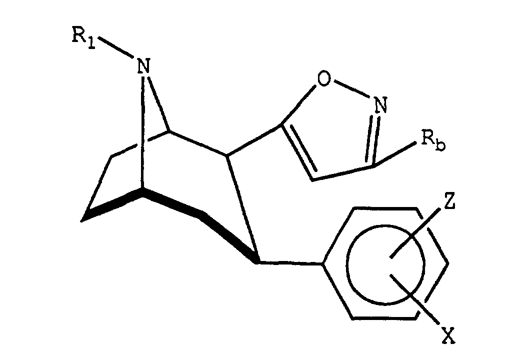 dans laquelle
R₁=hydrogène ou C₁₋₅ alkyle,
X=H, C₁₋₆ alkyle, C₃₋₈ cycloalkyle, C₁₋₄ alcoxy, C₁₋₆ alkynyle, halogène, amino ou acrylamido
Z=H, I, Br, Cl, F, CN, CF₃, NO₂, N₃, OR₁, CONH₂, CO₂R₁, C₁₋₆ alkyle, NR₄R₅, NHCOR₅, ou NHCO₂R₆, et
R_{b} est C₁₋₆ alkyle, phényle, ou phényle substitué par un C₁₋₆ alkyle.

26. Composition pharmaceutique pour traiter l'abus de psychostimulants, contenant une quantité pharmaceutiquement efficace du composé de formule : 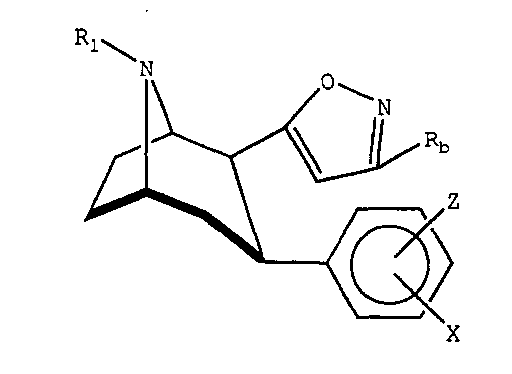 dans laquelle
R₁=hydrogène ou C₁₋₅ alkyle,
X=H, C₁₋₆ alkyle, C₃₋₈ cycloalkyle, C₁₋₄ alcoxy, C₁₋₆ alkynyle, halogène, amino ou acrylamido
Z=H, I, Br, Cl, F, CN, CF₃, NO₂, N₃, OR₁, CONH₂, CO₂R₁, C₁₋₆ alkyle, NR₄R₅, NHCOR₅, ou NHCO₂R₆, et
R_{b} est C₁₋₆ alkyle, phényle, ou phényle substitué par un C₁₋₆ alkyle.

27. Composition pharmaceutique pour inhiber l'action d'un psychostimulant, contenant une quantité du composé de formule : 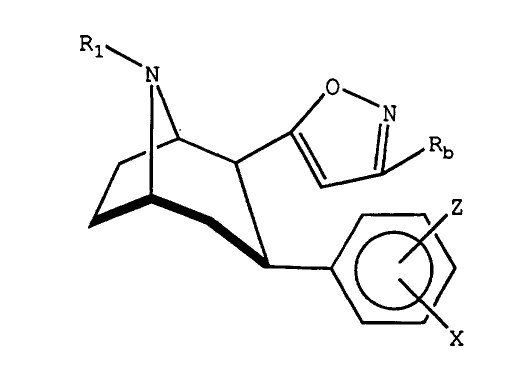 dans laquelle
R₁=hydrogène ou C₁₋₅ alkyle,
X=H, C₁₋₆ alkyle, C₃₋₈ cycloalkyle, C₁₋₄ alcoxy, C₁₋₆ alkynyle, halogène, amino ou acrylamido
Z=H, I, Br, Cl, F, CN, CF₃, NO₂, N₃, OR₁, CONH₂, CO₂R₁, C₁₋₆ alkyle, NR₄R₅, NHCOR₅, ou NHCO₂R₆, et
R_{b} est C₁₋₆ alkyle, phényle, ou phényle substitué par un C₁₋₆ alkyle,
apte à inhiber les psychostimulants.

28. Composition pharmaceutique pour inhiber le recaptage de neurotransmetteurs, contenant une quantité du composé de formule : 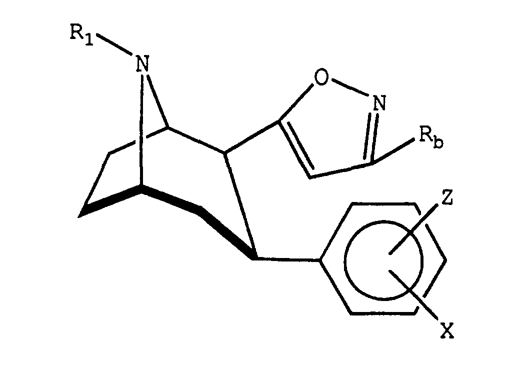 dans laquelle
R₁=hydrogène ou C₁₋₅ alkyle,
X=H, C₁₋₆ alkyle, C₃₋₈ cycloalkyle, C₁₋₄ alcoxy, C₁₋₆ alkynyle, halogène, amino ou acrylamido
Z=H, I, Br, Cl, F, CN, CF₃, NO₂, N₃, OR₁, CONH₂, CO₂R₁, C₁₋₆ alkyle, NR₄R₅, NHCOR₅, ou NHCO₂R₆, et
R_{b} est C₁₋₆ alkyle, phényle, ou phényle substitué par un C₁₋₆ alkyle,
apte à inhiber les transporteurs des neurotransmetteurs.

29. Composition pharmaceutique pour traiter les troubles neurodégénératifs, contenant une quantité pharmaceutiquement efficace du composé de formule : 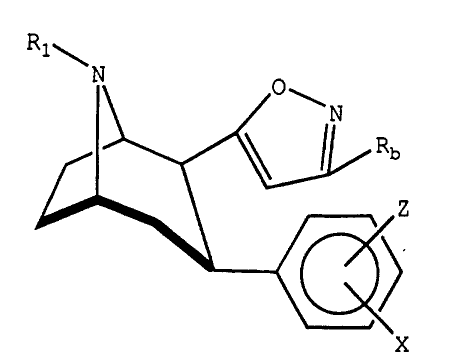 dans laquelle
R₁=hydrogène ou C₁₋₅ alkyle,
X=H, C₁₋₆ alkyle, C₃₋₈ cycloalkyle, C₁₋₄ alcoxy, C₁₋₆ alkynyle, halogène, amino ou acrylamido
Z=H, I, Br, Cl, F, CN, CF₃, NO₂, N₃, OR₁, CONH₂, CO₂R₁, C₁₋₆ alkyle, NR₄R₅, NHCOR₅, ou NHCO₂R₆, et
R_{b} est C₁₋₆ alkyle, phényle, ou phényle substitué par un C₁₋₆ alkyle.

30. Composition pharmaceutique pour traiter la dépression, contenant une quantité pharmaceutiquement efficace du composé de formule : 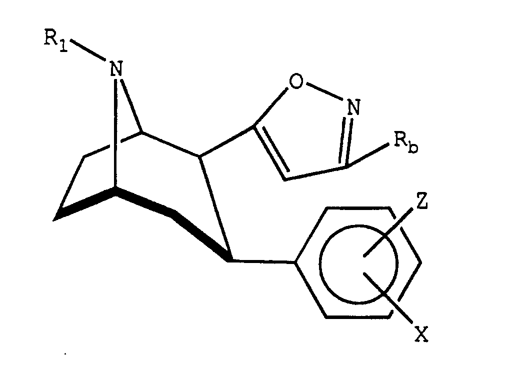 dans laquelle
R₁=hydrogène ou C₁₋₅ alkyle,
X=H, C₁₋₆ alkyle, C₃₋₈ cycloalkyle, C₁₋₄ alcoxy, C₁₋₆ alkynyle, halogène, amino ou acrylamido
Z=H, I, Br, Cl, F, CN, CF₃, NO₂, N₃, OR₁, CONH₂, CO₂R₁, C₁₋₆ alkyle, NR₄R₅, NHCOR₅, ou NHCO₂R₆, et
R_{b} est C₁₋₆ alkyle, phényle, ou phényle substitué par un C₁₋₆ alkyle.

31. Composition pharmaceutique pour traiter l'anorexie, contenant une quantité pharmaceutiquement efficace du composé de formule : 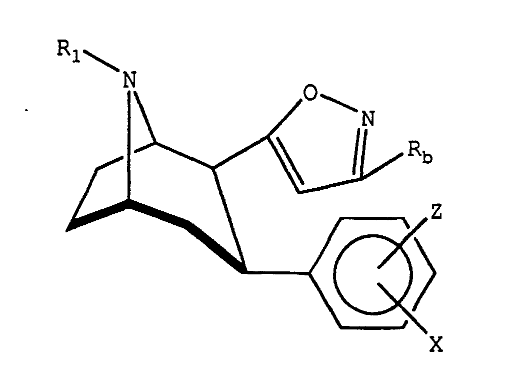 dans laquelle
R₁=hydrogène ou C₁₋₅ alkyle,
X=H, C₁₋₆ alkyle, C₃₋₈ cycloalkyle, C₁₋₄ alcoxy, C₁₋₆ alkynyle, halogène, amino ou acrylamido
Z=H, I, Br, Cl, F, CN, CF₃, NO₂, N₃, OR₁, CONH₂, CO₂R₁, C₁₋₆ alkyle, NR₄R₅, NHCOR₅, ou NHCO₂R₆, et
R_{b} est C₁₋₆ alkyle, phényle, ou phényle substitué par un C₁₋₆ alkyle.
